# EUROPÄISCHE PATENTSCHRIFT

(11) **EP 2 052 086 B1**
(45) Veröffentlichungstag und Bekanntmachung des Hinweises auf die Patenterteilung: **13.03.2013**
(21) Anmeldenummer: 07788393.2
(22) Anmeldetag: 13.08.2007
(51) Int. Cl.: C12Q 1/68

(54) **VERFAHREN ZUR SYNTHESE EINER cDNA IN EINER PROBE IN EINER ENZYMATISCHEN REAKTION**
METHOD FOR SYNTHESIS OF A cDNA IN A SAMPLE IN AN ENZYMATIC REACTION
PROCÉDÉ PERMETTANT LA SYNTHÈSE D'UN ADNC DANS UN ÉCHANTILLON PRÉSENT DANS UNE RÉACTION ENZYMATIQUE

(30) Priorität: 14.08.2006 DE 102006038113
(43) Veröffentlichungstag der Anmeldung: 29.04.2009
(73) Patentinhaber: Qiagen GmbH, 40724 Hilden (DE)
(72) Erfinder: ENGEL, Holger, 40724 Hilden (DE); YERRAMILLI, Subrahmanyam, Clarksville, Maryland 21029 (US); KREUTZ, Martin, 42697 Solingen (DE); LÖFFERT, Dirk, 40724 Hilden (DE); KORFHAGE, Christian, 40724 Hilden (DE)
(74) Vertreter: Kilger, Christian
(86) Internationale Anmeldenummer: PCT/EP2007/058369
(87) Internationale Veröffentlichungsnummer: WO 2008/020008

(56) Entgegenhaltungen:
- WO-A-2004/044239
- WO-A-2005/064019
- CN-A- 1 763 223
- US-A1- 2003 113 875
- US-A1- 2003 186 288
- US-B1- 6 300 069
- HELL A ET AL: "SYNTHESIS OF DNAS COMPLEMENTARY TO HUMAN RIBOSOMAL RNAS POLYADENYLATED IN VITRO" BIOCHIMICA ET BIOPHYSICA ACTA, AMSTERDAM, NL, Bd. 442, Nr. 1, 1976, Seiten 37-49, XP009045943 ISSN: 0006-3002
- KO ET AL: "RNA-conjugated template-switching RT-PCR method for generating an Escherichia coli cDNA library for small RNAs" JOURNAL OF MICROBIOLOGICAL METHODS, ELSEVIER, AMSTERDAM,, NL, Bd. 64, Nr. 3, März 2006 (2006-03), Seiten 297-304, XP005286712 ISSN: 0167-7012

## Beschreibung

### HINTERGRUND DER ERFINDUNG

Die Erfindung betrifft das Gebiet der Molekularbiologie sowie der Forschung in diesem Gebiet aber auch die humane sowie nicht-humane Diagnostik.

Die Analyse von nicht-polyadenylierten RNA-Molekülen wie zum Beispiel bakteriellen RNAs oder kleinen RNAs, wie den sogenannten microRNAs (miRNA's) gestaltet sich schwierig und erfordert spezielle Verfahren. Ein mögliches Verfahren wurde kürzlich in der Literatur beschrieben. Dieses Verfahren umfasst mehrere nacheinander geschaltete enzymatische Schritte, d.h. zuerst wird ein "Tailing" der RNA mit Poly-(A)-Polymerase und einem geeigneten Substrat, typischerweise ATP, durchgeführt. Danach wird die Poly-(A)-Reaktion gestoppt und das Reaktionsprodukt aufgereinigt. Anschließend wird die generierte Poly-(A)-RNA in eine reverse Transkriptasereaktion gegeben und mit geeigneten Primern in cDNA umgeschrieben.

### TECHNISCHES GEBIET

Die Durchführung dieser beiden nacheinander geschalteten enzymatischen Reaktionen ist aufwändig in der Durchführung und besitzt eine Vielzahl von Fehlerquellen, zum Beispiel Eintrag von Nukleasen, Verlust von Material oder Pipettierfehler.

microRNAs (miRNAs) variieren in der Größe von etwa 20 bis 25 Nukleotide und stellen eine neue Familie von nicht-kodierenden RNA's dar.

Sie werden über einen sogenannten "Hairpin Precursor" prozessiert und können als negative Regulatoren in der Genexpression eine Rolle spielen. So regulieren sie eine Vielzahl von Genen herunter (Ambros, V., 2001, MicroRNA's: Tiny regulators with great potential, Cell 107, 823-826). miRNAs werden zunächst als lange "primary transcripte" (sie werden auch primary miRNAs genannten) transkribiert (Lee, Y., Jeon, K. et al., 2002, MicroRNA maturation: stepwise processing in subcellular localisation, Embo J. 21, 4663-4670). Diese "primary transcripte" werden danach verkürzt, wobei die daraus resultierende Länge in etwa 70 Nukleotide beträgt. Es entstehen sogenannte "stem-loop structures", sie werden auch "prä-miRNAs" genannt. Prä-miRNAs werden in das Zytoplasma exportiert. Das exportierende Enzym nennt sich Exportin-5. Sie werden hier weiter prozessiert und es entsteht auf diese Weise ein etwa 22 Nukleotid langes, reifes miRNA-Molekül (Lee, Y., et al., 2003, The nuclear RNA's III Drosha initiates microRNA processing, Nature 425, 415-419). Jüngste Studien haben vorgeschlagen, dass miRNAs eine wichtige Rolle bei der Entwicklung und Differenzierung spielen. Grundsätzlich können microRNAs auf zwei verschiedene Arten und Weisen regulierend einwirken. In Pflanzen komplementieren miRNAs mit ihren korrespondierenden mRNAs durch exakte Komplementät. Dies führt zu einer Zerstörung der Ziel-mRNA durch einen Mechanismus, welcher RNA-Interferenz (RNAi) umfasst. In Tieren verhindern miRNAs Genexpression durch einen Mechanismus, welcher Lin-4 und Let-7 umfasst. Hier sind die miRNAs nicht genau komplementär zu ihren korrespondierenden mRNAs, jedoch verhindern sie die Synthese und Funktion der Proteine (Ambros, V., 2004, The functions of animal microRNAs, Nature, 431, 350-355). Aufgrund der entscheidenden Rolle, die die erst kürzlich entdeckten miRNAs spielen, kommt deren Nachweis bzw. Analyse eine entscheidende Rolle zu.

In Eukaryonten umfasst die Synthese der 18s, 5,8s und 25/28s rRNAs die Prozessierung in Modifikationen sogenannten Precursor-rRNAs (pre-rRNA) im Nukleolus. Dieser komplexe Ablauf der rRNA-Biogenese umfaßt viele kleine sogenannte "small nucleolar RNAs" (snoRNA) welche sich im Nukleolus anreichern. Sie tun dies in Form sogenannter small nucleolar ribonucleo protein particles (snoRNPs) (Maxwell, E. S. et. al., 1995, The small nucleolar RNAs, Annual Review Biochem., 35, 897-934).

Alle bis dato charakterisierten snoRNAs, mit der Ausnahme der RNase MRP fallen in zwei Familien. Diese sind die box c/D und box h/ACA slow RNAs, die sich durch ihnen gemeinsame Sequenzmotive unterscheiden lassen (Ballakin, A. D. et al., 1996, The RNA world of the nucleolus: two major families of small nucleolar RNAs defined by different box elements with related functions, Cell, 86, 823-834). Die genomische Organisation der snoRNA-Gene weist eine große Diversität in verschiedenen Eukaryonten auf. In Vertebraten sind die meisten snoRNAs innerhalb von Introns via "host gene" eingefügt. Ausnahmen wie U3 werden unabhängig transkribiert. In Hefe gibt es snoRNAs welche in Introns eingefügt sind, jedoch wird die Majorität der snoRNAs als Einzelgen mit einem eigenen Promotor transkribiert. Geklusterte snoRNA-Gene werden durch gemeinsame Promotoren upstream transkribiert. Aufgrund der kleinen Größen und der fehlenden Polyadenylierung ist der Nachweis bzw. die Analyse von snoRNAs eine molekularbiologische Herausforderung.

Die PCR ist ein häufig eingesetztes Instrument zur Studie mikrobieller Organismen und wird mit unter auch verwendet um 16S rRNA-Gene zu analysieren. Jedoch ist die Entdeckung neuer Gene in mikrobiellen Proben eingeschränkt durch die nur bedingt mögliche Synthese von Primern. So werden Primer für 16S RNA-Gene von jenen Sequenzen abgeleitet, die man von kultivierten Mikroben bereits kennt (Olson, D. J., 1986, Microbial ecology and evolution: A ribosomal RNA approach, Annu. Rev. Microbial. 40: 337-365). Aufgrund der Systematik, dass man nämlich für die Extraktion von 16S rRNA-Genen aus bis dato unbekannten Organismen auf Sequenzen zurückgreift, die man bereits kennt, ist es wahrscheinlich, daß die mikrobielle Diversität stark unterschätzt und auch nicht isoliert wird.

Ebenso wie die 16S rRNA-Moleküle nur schwer zu isolieren sind, sind prokaryontische mRNA-Moleküle mangels Kenntnis der Sequenz und insbesondere mangels Poly-A-Schwanz nur schwer zu isolieren.

Der Stand der Technik kennt einen 2-stufigen Prozess. Bei diesem Verfahren wird ein RNA-Molekül unter zu Hilfenahme des Enzyms Poly-A-Polymerase und dem Substrat Adenosintriphosphat so umgesetzt, dass ein polyadenyliertes Ribonukleinsäuremolekül entsteht. Dieses so polyadenylierte Ribonukleinsäuremolekül wird in einem weiteren Schritt aufgereinigt, bevor in einem dritten Schritt eine reverse Transkription stattfindet. Die reverse Transkription macht sich den polyadenylierten Schwanz zueigen, wobei ein homopolymeres Oligonukleotid in der Regel ein Poly-T-Oligonukleotid den polyadenylierten RNA-Schwanz komplementär anlagert. Das 3'-Ende des Poly-T-Oligonukleotids wird nun von der Polymerase genutzt, um einen Desoxyribonkleinsäurestrang zu erstellen, der komplementär zu dem vorliegenden Ribonukleinsäurcstrang ist. Der so entstandene Strang nennt sich "first strand cDNA". Diese cDNA kann in einer PCR-Reaktion genutzt werden, wobei es zum Einsatz von entweder Random-Primem oder aber spezitischen Primern kommt um so ein Amplifikat zu generieren. Shi *et*. *al* lehrt speziell den miRNA Nachweis über einen Oligo-dT Adapter-Primer, wobei ein Adapter spezifischer Primer in der PCR eingesetzt wird (Shi, R. and Chiang, V. L. (Shi, R. et al., Facile means for quantifying microRNA expression by real-time PCR, Biotechniques, 2005, 39, 519-25). Ähnliche Verfahren sind aus US 2003186288 A1 sowie US 6 300 069 B1 bekannt. Dieses erst jüngst publizierte Verfahren hat entscheidende Nachteile in Bezug auf die oben genannten speziellen Ribonukleinsäuremoleküle.

So bedingt das zweistufige Verfahren allgemein möglicherweise einen Eintrag von Verunreinigungen. Der Aufreinigungsschritt führt zu Verlusten von seltenen RNAs. Das zweistufige Verfahren erfordert eine Inaktivierung des ersten Enzyms sowie eine Inkubationszeit für das erste und das zweite Enzym, was insgesamt zu einem sehr großen Zeitaufwand führt. Das zweistufige Verfahren hat weiterhin den Nachteil, dass eine Verwechslungsgefahr von Proben dann auftreten kann, wenn zwei oder mehr Proben gleichzeitig bearbeitet werden. Wie aus dem Stand der Technik bekannt ist, sind Ribonukleinsäuren relativ empfindlich was den Angriff von Nukleasen anbelangt. Das zweistufige Verfahren insbesondere der Schritt der Aufreinigung nach dem ersten Verfahren birgt die Gefahr, dass Nukleasen eingebracht werden. Letztendlich führen zwei oder mehr Schritte stets dazu, dass die Gefahr von Pipettierfehlem steigt.

### GEGENSTAND DER ERFINDUNG

Es ist somit Aufgabe der vorliegenden Erfindung ein Verfahren bereitzustellen, welches die cDNA-Synthese ermöglicht, Verunreinigungen weitestgehend verhindert, weniger zeitaufwändig ist, die Gefahr von Verwechslung der Proben minimiert, die Gefahr des Eintrags von Nukleasen minimiert und schlussendlich die Gefahr von Pipettierfehlem weitestgehend ausschließt.

Die erfindungsgemäße Lösung dieser Aufgabe ist in den Ansprüchen definiert. Die vorliegende Aufgabe wird gelöst durch ein Verfahren zur Synthese einer cDNA in einer Probe, in einer enzymatischen Reaktion, wobei das Verfahren folgende Schritte umfasst:
(a) gleichzeitige Bereitstellung eines ersten Enzyms mit Polyadenylierungsaktivität, eines zweiten Enzyms mit reverser Transkriptaseaktivität, eines Puffers, mindestens eines Ribonukleotids, mindestens eines Desoxyribonukleotids, eines Anker Oligonukleotids, (b) Zugabe einer Probe umfassend eine Ribonukleinsäure und (c) Inkubation der Agenzien der Schritte (a) und (b) bei einem oder mehreren Temperaturschritten, welche so gewählt sind, dass das erste und das zweite Enzym Aktivität zeigen.

Bis dato bestanden Vorbehalte, dass eine Kombination der enzymatischen Polyadenylierung und der reversen Transkription technisch möglich ist. Dies zeigt sich daran, dass selbst in jüngster Zeit, also nach Entdeckung von microRNAs und snoRNAs, die eine besondere molekularbiologische Herausforderung in Bezug auf Analyse und Isolierung darstellen, die enzymatischen Reaktionen stets nacheinander durchgeführt wurden (Want, J. F., et al., Identification of 20 microRNAs from Oryza sativa, Nucleic Acid Res, 2004, 32, 1688-95; Shi, R. and Chiang, V. L., Facile means for quantifying microRNA expression by realt-time PCR, Biotechniques, 2005, 39, 519-25; Fu H., et al., Identification of human fetal liver miRNAs by a novel method; FEBS Lett, 2005, 579, 3849-54; Chen, C. L. et al., The high diversity of snoRNAs in plants: identification and comparative study of 120 snoRNA genes from Oryza sativa, Nucleic Acids Res, 2003, 31, 2601-13; Botero, L. M. et al., Poly(A) polymerase modification and reverse transcriptase PCR amplification of environmental RNA, Appl. Environ Microbiol, 2005, 71, 1267-75). Erstaunlicherweise sind beide Verfahren, d.h. also die Polyadenylierung und die reverse Transkription dem Fachmann schon lange bekannt (Sano, H. and Feix, G., Terminal riboadenylate transferase from Escherichia coli. Characterization and application, Eur. J. Biochem., 1976, 71, 577-83). Der Fachmann hat in der Regel nach dem Poly-A-Tailing-Schritt das Reaktionsprodukt aufgereinigt (Shi, R. et al., Facile means for quantifying microRNA expresssion by real-time PCR, Biotechniques, 2005, 39, 519-25). Der Grund dafür liegt sowohl in dem sich deutlich unterscheidenden Zusammensetzungen der Reaktionspuffer als auch den für die Reaktion benötigten Substraten.

Außerdem wird ein Verfahren beschrieben, welches die cDNA-Synthese ermöglicht, und diese Reaktion optional mit einer dritten enzymatischen Reaktion koppelt, welche den spezifischen Nachweis der generierten cDNA im gleichen Reaktionsgefäß erlaubt. Dieses "3-in-1" Verfahren zeigt durch seine sehr einfache Handhabung besondere Vorteile, wenn große Probenzahlen auf einen oder wenige Analyten hin analysiert werden sollen. Der Grund ist dass es, z.B. gekoppelt mit einer real-time PCR, ein sehr einfaches und schnelles Verfahren darstellt um große Zahlen von Proben zu analysieren. Zusätzliche Handhabungsschritte und Verunreinigungen werden so weitestgehend verhindert, wodurch es weniger zeitaufwändig ist, die Gefahr von Verwechslung der Proben minimiert, die Gefahr des Eintrags von Nukleasen minimiert und schlussendlich die Gefahr von Pipettierfehlem weitestgehend ausschließt.

Die Aufgabe der "3in1" Reaktion wird gelöst durch ein Verfahren zur Synthese einer cDNA in einer Probe, in einer enzymatischen Reaktion, gefolgt von einer weiteren enzymatischen Reaktion, optional einer Amplifikation, optional gekoppelt mit dem Nachweis, entweder in real-time während der Amplifikation oder nachgeschaltet, wobei das Verfahren folgende Schritte umfasst:
(a) gleichzeitige Bereitstellung eines ersten Enzyms mit Polyadenylierungsaktivität, eines zweiten Enzyms mit reverser Transkriptaseaktivität, eines Puffers, mindestens eines Ribonukleotids, mindestens eines Desoxyribonukleotids, eines Anker Oligonukleotids, mindestens eines dritten Enzyms mit Nukleinsäure-Synthese Aktivität, mindestens eines Primers, optional einer Sonde (b) Zugabe einer Probe umfassend eine Ribonukleinsäure und (c) Inkubation der Agenzien der Schritte (a) und (b) bei einem oder mehreren Temperaturschritten, welche so gewählt sind, dass das erste und das zweite Enzym Aktivität zeigen und optional das dritte Enzym aktiv oder inaktiv ist. Optional folgen einer oder mehrere Temperaturschritte, bei denen das erste und zweite Enzym wenig aktiv oder inaktiv sind und das dritte Enzym aktiv ist.

Das *in vivo* verwendete Substrat der Poly-(A)-Polymerase ist Adenosintriphosphat (ATP). Für einige Poly-(A)-Polymerasen wurde gezeigt, dass auch das Anfügen kurzer Tails mit anderen NTPs als Substrat möglich sein kann (Martin, G. und Keller, W., Tailing and 3'-end labeling of RNA with yeast poly(A) polymerase and various nucleotides, RNA, 1998, 4, 226-30).

Überaschenderweise haben die Erfinder der vorliegenden Erfindung herausgefunden, dass es möglich ist, unter bestimmten Voraussetzungen die beiden doch sehr unterschiedlichen enzymatischen Reaktionen gleichzeitig, in einem Reaktionsgefäß ablaufen zu lassen. In einer bevorzugten Ausführungsiform der Erfindung handelt es sich bei der Probe um eine Ribonukleinsäure, die ausgewählt ist aus der Gruppe umfassend prokaryontische Ribonukleinsäuren, eurkaryontische Ribonukleinsäuren, virale Ribonukleinsäuren, Ribonukleinsäuren deren Ursprung ein Archae-Organismus ist, microRibonukleinsäuren (miRNA), small nucleolar Ribonukleinsäuren (snoRNA), messenger Ribonukleinsäure (mRNA), Transfer-Ribonukleinsäuren (tRNA), nicht-polyadenylierte Ribonukleinsäuren im allgemeinen, sowie ribosomale Ribonukleinsäuren (rRNA) Darüber hinaus eine Mischung von zwei oder mehr der genannten Ribonukleinsäuren In der Probe kann natürlich auch bereits poly-A RNA enthalten sein.

In einer besonders bevorzugten Ausführungsform der vorliegenden Erfindung handelt es sich bei der Probe um eine Ribonukleinsäure, die ausgewählt ist aus der Gruppe, umfassend prokaryontische Ribonukleinsäuren, miRNA, snoRNA und rRNA. In der bevorzugtesten Ausführungsform der vorliegenden Erfindung umfasst die Probe eine Ribonukleinsäure, die ausgewählt ist aus der Gruppe umfassend miRNA und snoRNA. Bevorzugt werden weiter Mischproben aus unterschiedlichen Mengen von Ribonukleinsäuren unterschiedlicher Art einhergehend mit anderen Stoffen.

Zusätzlich wird hierin beschrieben, dass es möglich ist, unter bestimmten Voraussetzungen die beiden doch sehr unterschiedlichen enzymatischen Reaktionen gleichzeitig, in einem Reaktionsgefäß ablaufen zu lassen sowie dies zusätzlich mit einer dritten enzymatischen Reaktion zum spezifischen Nachweis der generierten cDNA zu koppeln, welche bevorzugt eine Nukleinsäure-Synthese Aktivität ist. In einer bevorzugten Ausführungsform der Erfindung handelt es sich bei der Probe um eine Ribonukleinsäure, die ausgewählt ist aus der Gruppe umfassend prokaryontische Ribonukleinsäuren, eukaryontische Ribonukleinsäuren, virale Ribonukleinsäuren, Ribonukleinsäuren deren Ursprung ein Archae-Organismus ist, microRibonukleinsäuren (miRNA), small nucleolar Ribonukleinsäuren (snoRNA), messenger Ribonukleinsäure (mRNA), Transfer-Ribonukleinsäuren (tRNA), nicht-polyadenylierte Ribonukleinsäuren im allgemeinen, sowie ribosomale Ribonukleinsäuren (rRNA) Darüber hinaus eine Mischung von zwei oder mehr der genannten Ribonukleinsäuren In der Probe kann natürlich auch bereits poly-A RNA enthalten sein.

In einer besonders bevorzugten Ausführungsform der vorliegenden Erfindung handelt es sich bei der Probe um eine Ribonukleinsäure, die ausgewählt ist aus der Gruppe, umfassend prokaryontische Ribonukleinsäuren, miRNA, snoRNA und rRNA. In der bevorzugtesten Ausfiihrungsform der vorliegenden Erfindung umfasst die Probe eine Ribonukleinsäure, die ausgewählt ist aus der Gruppe umfassend miRNA und snoRNA. Bevorzugt werden weiter Mischproben aus unterschiedlichen Mengen von Ribonukleinsäuren unterschiedlicher Art einhergehend mit anderen Stoffen.

Aufgrund der vorliegenden Vorteile des erfindungsgemäßen Verfahrens konnten die Erfinder zeigen, dass es möglich ist, miRNAs effizient und ohne Kontamination bereitzustellen und zu charakterisieren.

In einer Ausführungsform der Erfindung handelt es sich bei dem Anker-Oligonukleotid um ein homopolymeres Oligonukleotid, welches ausgewählt ist aus der Gruppe umfassende ein Poly-(T)-Oligonukleotid, oder ein Poly-(T)-Oligonukleotid zusätzlich umfassend einen 5'-Tail Bevorzugt wird ein Poly-(T)-Oligonukleotid, welches optional wie bereits oben ausgeführt, zusätzlich einen 5'-Tail haben kann.

Das erfindungsgemäße Anker-Oligonukleotid ist in der Regel zwischen 6 und 75 Nukleotide lang. Es kann jedoch bis zu ca. 150 Nukleotide lang sein. Ist das Anker Oligonukleotid synthetisch, so ergibt sich die maximale Länge aus den technischen Limitationen der DNA Synthese. Optional umfasst das Anker Oligonukleotid einen 5'-Tail und/oder eine Ankersequenz. Ein 5'-Tail ist eine zusätzliche Nukleotidsequenz am 5'-Ende des Oligonukleotids, die beispielsweise dazu dient eine Klonierungssequenz, Primer- und/oder Sonden-Bindungsstellen oder eine beliebige andere Sequenz einzuführen. Das identifizieren von geeigneten Sequenzen für den 5'-Tail ist für den Fachmann basierend auf den Anforderungen der jeweilige Anwendung möglich.

Am 3'-Ende des Anker-Oligonukleotids kann eine zusätzliche Ankersequenz, typischerweise mit einer Länge von ein bis fünf weiteren Nukleotiden, enthalten sein. Die Ankersequenz kann eine Länge von mindestens einer Base aufweisen, wobei die erste Position in einer bevorzugten Ausführungsform eine degenerierte Base ist, die alle Basen außer der im homopolymer Anteil des Anker-Oligonukleotids verwendeten Base enthält. Hierauf können weitere Basen folgen. Diese können auch degeneriert sein. In einer bevorzugten Ausführungsform ist hier die Verwendung von N. Wobbles sinnvoll, wobei N= A, C, G, T oder entsprechende Analoga.

In aller Regel ist das Anker-Oligonukleotid eine Desoxyribonukleinsäure (DNA). Es kann sich bei den Anker-Oligonukleotid jedoch auch um eine Peptidnukleinsäure (PNA) handeln. Möglich sind auch locked Nukleinsäuren (LNA), Phosphorthioat-Desoxyribonukleinsäuren, Cyclohexen-Nukleinsäuren (CeNA), N3'-P5'-Phosphoramedite (NP), Tricyklo-Desoxyribonukleinsäuren (tcDNA). Bevorzugt wird jedoch ein Anker-Oligonukleotid, welches eine Desoxyribonukleinsäure (DNA) ist. Möglich sind Mischungen von RNA und DNA oder einer oder mehrerer der modifizierten Nukleinsäuren oder Analoga, sowie andere Modifikationen wie entsprechende Basenanaloga, die unter den gewählten Bedingungen in der Lage sind mit RNA oder DNA zu hybridisieren. In einer besonders bevorzugten Ausführungsform handelt es sich bei dem Anker Oligonukleotid um ein Poly-(T)-Oligonukleotid, welches zusätzlich ein 5'-Tail umfasst, eine Desoxyribonukleinsäure ist, 15-150 Nukleotide lang ist und als Gemisch vorliegt, Am 3'-Ende des Anker Oligonukleotids kann eine zusätzliche Ankersequenz typischerweise mit einer Länge von ein bis fünf weitere Nukleotiden enthalten sein. Die Ankersequenz kann eine Länge von mindestens einer Base aufweisen, wobei die erste Position in einer bevorzugten Ausführungsform eine degenerierte Base ist, die alle Basen außer der im homopolymer Anteil des Anker Oligonukleotids verwendeten Base enthält. Hierauf können weitere Basen folgen. Diese können auch degeneriert sein. In einer bevorzugten Ausführungsform ist hier die Verwendung von N Wobbles sinnvoll, wobei N= A, C, G, T oder entsprechende Analoga.

Beispielhaft seien folgende erfindungsgemäße Anker Oligonukleotide genannt:
Beispiel 1 (SEQ ID NO: 10): 5' TGG AAC GAG ACG ACG ACA GAC CAA GCT TCC CGT TCT CAG CC (T)ₓ VVN 3'
Beispiel 2 (SEQ ID NO: 11): 5' AACGAGACGACGACAGAC(T)ₓVN 3'
Beispiel 3 (SEQ ID NO: 12): 5' AACGAGACGACGACAGAC(T)ₓV 3'
Beispiel 4 (SEQ ID NO: 13): 5' AACGAGACGACGACAGAC(T)ₓN 3'
Beispiel 5 (SEQ ID NO: 14): 5' AACGAGACGACGACAGAC(T)ₓNN 3'
Beispiel 6 (SEQ ID NO: 15): 5' AACGAGACGACGACAGAC(T)ₓVNN 3'
Beispiel 7 (SEQ ID NO: 16): 5' AACGAGACGACGACAGAC(T)ₓVNNN 3'
Beispiel 8 (SEQ ID NO: 17): 5' AACGAGACGACGACAGAC(T)ₓNNN 3'
Beispiel 9 (SEQ ID NO: 18) : 5' TGG AAC GAG ACG ACG ACA GAC CAA GCT TCC CGT TCT CAG CC(T)ₓVN 3'
Beispiel 10 (SEQ ID NO: 19): 5' TGG AAC GAG ACG ACG ACA GAC CAA GCT TCC CGT TCT CAG CC(T)ₓVNN 3'
X ist bevorzugt 10 bis 30 Basen.
V und N sind aus dem Single Letter Code für degenerierte Basen, V= A, C, G; N= A, C, G, T.

Die Identifikation anderer geeigneter 5' Tail Sequenzen ist dem Fachmann möglich.

Der optionale 5'-Tail umfasst zusätzliche 1-100 Nukleotide, welche für nachfolgende Analysen Verwendung finden können. So kann in einer bevorzugten Ausführungsform der 5'Tail die Bindungssequenz für ein Oligonukleotid wie z.B. eine oder mehrere DNA Sonden und/oder einen oder mehrere PCR Primer enthalten. Die verwendeten Sequenzen für den 5'Tail werden bevorzugt so ausgewählt, dass diese mit dem erfindungsgemäßen Verfahren kompatibel sind. Dies umfasst z.B. die Auswahl solcher Sequenzen, die keine ungewünschten Nebenreaktionen, sowohl im erfindungsgemäßen Verfahren als auch bei nachfolgen Analyseverfahren, hervorrufen.

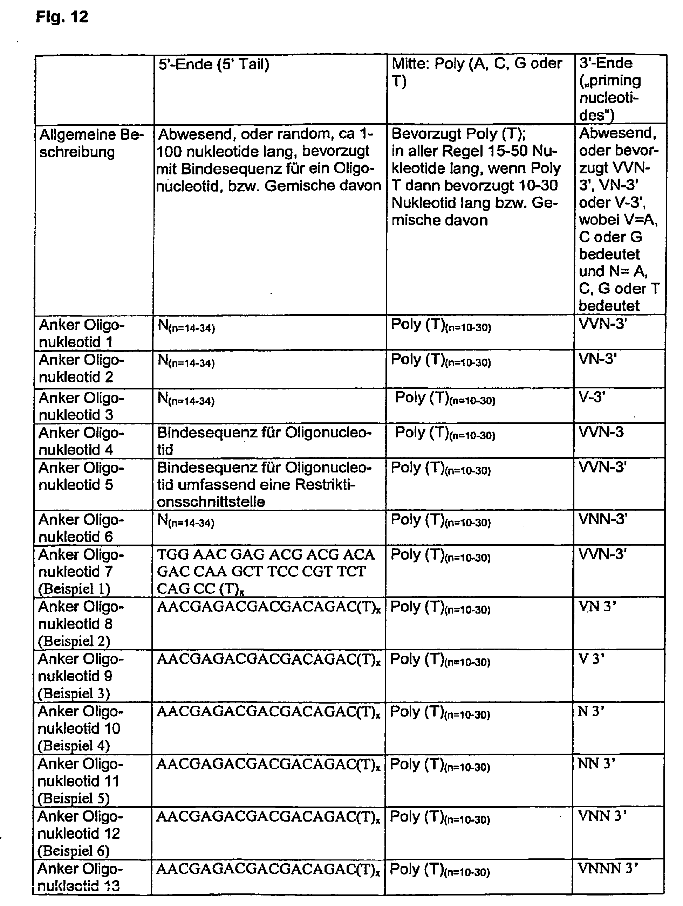

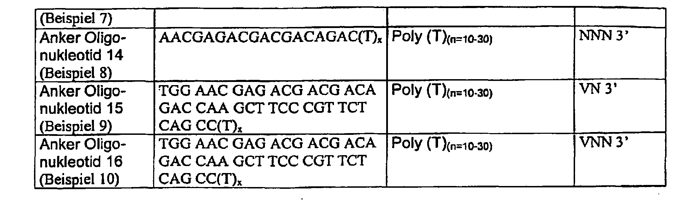

Erfindungsgemäße Anker Oligonukleotide sind in Fig. 12 gezeigt.

Grundsätzlich kann die erfindungsgemäße enzymatische Reaktion auf einem Träger bzw. in einem Behältnis stattfinden, d.h. die Reaktion kann in einem Reaktionsgefäß stattfinden. Ein solches Reaktionsgefäß kann ein Reaktionsröhrchen oder beispielsweise eine Microtiterplatte sein. Die Reaktion kann auf einem Chip stattfinden. Findet sie auf einem Chip statt, können eine oder mehrere Komponenten immobilisiert sein. Die Reaktion kann auf einem Teststreifen oder in einem mikrofluidischen System stattfinden. Dem Fachmann sind verschiedenste Ausführungsformen bezüglich des Trägers bzw. Behältnisses bekannt.

In einer bevorzugten Ausführungsform handelt es sich bei dem Ribonukleotid um ein Adenosin-5'-Triphosphat, ein Thymidin-5'-Triphosphat, ein Cytosin-5'-Triphosphat, eine Guanin-5'-Triphosphat und/oder ein Uracil-5'-Triphosphat. Das Ribonukleotid kann auch ein Basenanalogon sein. Das Ribonukleotid kann modifiziert oder markiert sein. Grundsätzlich ist wesentlich, dass das Ribonuklcotid von dem Enzym im Polyadenylierungsaktivität als Substrat umgesetzt werden kann.

Das erfindungsgemäße Desoxyribonukleotid kann ausgewählt sein aus der Gruppe umfassend, ein Desoxyadenosin-5'-Triphosphat (dATP), ein Desoxythymin-5'-Triphosphat (dTTP), ein Desoxycytosin-5'-Tripliosphat (dCTP), ein Desoxyguanosin-5'-Triphosphat (dGTP), Desoxyuracil-5'-Triphosphat (dUTP) sowie modifizierte Desoxyribonukleotide und markierte Desoxyribonukleotide. Es sind auch Anwendungen denkbar, bei denen zusätzlich oder im Austausch ein oder mehrere Desoxyribonukleotide, die eine universelle Base oder ein Basenanalogon enthalten, eingesetzt werden. Wesentlich für die Durchführung der Erfindung ist, dass die verwendeten Desoxyribonukleotide eine cDNA Synthese erlauben.

Erfindungsgemäß bevorzugt ist es, wenn dATP, dCTP, dTTP und dGTP als Gemisch gemeinsam vorliegen.

Erfindungsgemäß kann in dem Gemisch auch Desoxyuracil-5'-Triphosphat verwendet werden. Dies kann kombiniert werden mit einer nach der eigentlichen Reaktion stattfindenden enzymatischen Reaktion, welche die Uracil-DNA-Glykosilase verwendet und nicht weiter genutztes enzymatisch hergestelltes Reaktionsprodukt abbauen kann.

Sofern ein Desoxyribonukleotid markiert ist, kann die Markierung ausgewählt werden aus der Gruppe umfassend ³²P, ³³P, ³⁵S, ³H, ein fluoreszierender Farbstoff wie beispielsweise Fluoresceinisothiocyanate (FITC), 6-Carboxyfluorescein (FAM), Xanthen, Rhodamine, ,6-Carboxy-2',4',7',4,7-hexachlorofluorescein (HEX), 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine 110; Coumarine, wie Umbelliferone, Benzimide, wie Hoechst 33258; Phenanthridine, wie Texas Red, Ethidiumbromide, Acridinfarbstoffe, Carbazolfarbstoffe, Phenoxazinefarbstoffe, Porphyrinfarbstoffe, Polymethinfarbstoffe, Cyaninfarbstoffe, wie Cy3, Cy5, Cy7, BODIPY-Farbstoffe, Quinolinfarbstoffe und Alexa Farbstoffe. Andere Markierungen wie das Einfügen von Biotin oder einem oder mehreren Haptenen wie z.B. Digoxigenin, welchen einen direkten oder indirekten Nachweis der Nukleinsäure erlauben. Indirekte Nachweise wie z.B. über Antikörper, welche wiederum einen z.B. enzymatischen Nachweis über ein am Antikörper gekoppeltes Enzym. Auch über das Einbringen von Nanopartikeln, die z.B. an Antikörper oder einen Affinitätsliganden gekoppelt sind, ist ein indirekter Nachweis möglich.

Eine Modifikation des Desoxyribonukleotids kann auch über das 5'-Phosphat erfolgen, welches eine einfachere Klonierung erlaubt. Durch einfügen von reaktiven Gruppen, wie z.B. einem Amino-Linker (auch Biotin) kann das Desoxyribonukleotid z.B. immobilisiert werden oder einem direkten oder indirekten Nachweis zugänglich gemacht werden.

Besonders bevorzugte Modifikationen sind ausgewählt aus der Gruppe umfassend Fluoreszenfarbstoffe, Haptene, 5'-Phosphat, 5'- Biotin, 5'-Amino-Linker.

Erfindungsgemäß ist die Konzentration eines Desoxyribonukleotids in der Reaktion wenigstens 0,01 mM und höchstens 10 mM. Bei dieser Konzentrationsangabe handelt es sich um die Konzentration des einzelnen Desoxyribonukleotids. In einer der bevorzugten Ausführungsformen liegen die Desoxyribonukleotide jeweils dATP, dCTP, dGTP und dTTP in einer Konzentration von 0,2 mM bis 2 mM vor. Bei dieser Konzentrationsangabe handelt es sich um die Konzentration des einzelnen Desoxyribonukleotids im Gemisch. In einer besonders bevorzugten Ausführungsform der Erfindung liegt das einzelne Desoxyribonukleotid, dATP, dCTP, dGTP und dTTP in einer Konzentration von jeweils 0,5 mM vor.

Die Erfinder haben erstaunlicherweise festgestellt, dass die Ein-Schritt Enzymreaktion wie sie Gegenstand der vorliegenden Erfindung ist, in einem engen Puffer-pH-Bereich von 6 bis 10 unter Anwesenheit von Magnesium Ionen (Mg²⁺) stattfinden kann. Somit liegt in einer bevorzugten Ausführungsform ein pH von 6 bis 10 vor.

In einer besonders bevorzugten Ausführungsform hat der erfindungsgemäße Puffer einen pH von 6,8 bis 9.

In einer weiteren Ausführungsform der erfindungsgemäßen Erfindung umfasst der erfindungsgemäße Puffer zusätzlich Ionen, die ausgewählt sein können aus der Gruppe umfassend, Mn²⁺, K⁺, NH⁴⁺ und Na⁺.

Erfindungsgemäße Puffer beinhalten beispielsweise MgCl₂, MgSO₄, Magensiumacetat, MnCl₂, KCI, (NH₄)₂SO₄, NH₄Cl, NaCl. Als Puffersubstanz kommen in Frage, Tris, Tricine, Bicine, Hepes, sowie andere Puffersubstanzen die im erfindungsgemäßen pH-Bereich liegen oder Mischungen von zwei oder mehr geeigneten Puffersubstanzen.

Dem Fachmann sind eine Reihe von Enzymen mit Polyadenylierungsaktivität bekannt. Erfindungsgemäß sind diese ausgewählt aus der Gruppe umfassend, Enzyme prokaryontischen Ursprungs, eukaryontischen Ursprungs, viralen Ursprungs und archae Ursprungs sowie auch Enzyme pflanzlichen Ursprungs.

Eine Polyadenylierungsaktivität im Sinne dieser Erfindung ist eine enzymatische Aktivität, die als Substrat das 3'-Ende einer Ribonukleinsäure verwendet und in einem geeigneten Puffer in der Lage ist, diesem 3'-Ende enzymatisch Ribonukleotide hinzuzufügen und zwar bevorzugterweise wenigstens 10 bis 20 Ribonukleotide. In einer bevorzugten Ausführungsform handelt es sich bei dem Enzym um ein Enzym, welches in der Lage ist, Adenosin-5'-Triphosphat als Substrat zu verwenden. Erfindungsgemäß umfasst dies Enzyme und Reaktionsbedingungen, die eine Polyadcnylierungs-aktivität im Sinne der Erfindung bei Verwendung von einzelsträngiger als auch doppelsträngige RNA, z.B. hairpin RNA wie, z.B. pre-miRNA aufweisen. In Abhängigkeit von der zu analysierenden RNA wird der Fachmann Enzym und Reaktionsbedingungen so auswählen, dass entweder einzelsträngige RNA's (z.B. reife miRNA's) oder doppelsträngige RNA's (z.B. prä-miRNA's) oder beides der Analyse zugänglich gemacht werden.

Eine Polyadenylierungsaktivität im Sinn der Erfindung ist allgemein eine Transkriptaseaktivität.

In einer bevorzugten Ausführungsform handelt es sich bei dem Enzym mit Polyadenylierungsaktivität um ein Enzym, welches ausgewählt ist aus der Gruppe umfassend, Poly-(A)-Polymerase aus *Escherichia coli.,* Poly-(A)-Polymerase aus Hefe, Poly-(A)-Polymerase aus Rind, Poly-(A)-Polymerase aus Frosch, humane Poly-(A)-Polymerase, und pflanzliche Poly-(A)-Polymerase. Weitere sind dem Fachmann bekannt oder durch die Analyse der Homologie zu bekannten Poly-(A)-Polymerasen neu zu identifizieren. In einer besonders bevorzugten Ausführungsform handelt es sich bei dem Enzym mit Polyadenylierungsaktivität um eine Poly-(A)-Polymerase aus *Escherichia coli.*

Das erfindungsgemäße Enzym mit reverser Transkriptaseaktivität ist erfindungsgemäß ausgewählt aus der Gruppe umfassend, Enzyme aus Viren, Bakterien, Archae-Bakterien und Eukaryonten, insbesondere aus thermostabilen Organismen. Hierzu zählen z.B. auch Enzyme aus Introns, Retrotransposons oder Retroviren. Ein Enzym mit reverser Transkriptaseaktivität ist erfindungsgemäß ein Enzym, welches in der Lage ist, an einer Ribonukleinsäure am 3'-Ende eines an die Ribonukleinsäure-hybridisierten Desoxyoligonukleotides oder Ribooligonukleotides bei geeigneten Pufferbedingungen Desoxyribonukleotide komplementär einzubauen. Dies umfasst zum einen Enzyme, die natürlicherweise diese Funktion aufweisen aber auch Enzyme, die eine solche Funktion erst durch Veränderung ihrer Gensequenz wie z.B. Mutagenese oder durch entsprechende Pufferbedingungen erhalten.

Bevorzugt ist das Enzym mit reverser Transkriptaseaktivität, ein Enzym, welches ausgewählt ist aus der Gruppe umfassend HIV reverse Transkriptase, M-MLV reverse Transkriptase, EAIV reverse Transkriptase, AMV reverse Transkriptase, *Thermus thermophilus* DNA Polymerase I, M-MLV RNAse H, Superscript, Superscript II, Superscript III, Monstersript (Epicentre), Omniscript, Sensiscript Reverse Transkriptase (Qiagen), ThermoScript und Thermo-X (beide Invitrogen). Erfindungsgemäß können auch Enzyme verwendet werden, die erst nach einer Modifikation der Gensequenz als Enzym reverse Transkriptaseaktivität aufweisen. Es kann auch eine Reverse Transkriptaseaktivität verwendet werden, die eine erhöhte Fehlergenauigkeit aufweist. Beispielhaft sei hier z.B. AccuScript reverse Transcriptase (Stratagene) erwähnt. Es ist für den Fachmann ersichtlich, dass auch die Verwendung von Mischungen von zwei oder mehr Enzymen mit reverser Transkriptaseaktivität möglich ist.

Dem Fachmann ist bekannt, dass die meisten Enzyme mit reverser Transkriptaseaktivität ein divalentes Ion benötigen. Somit liegt einer bevorzugten Ausführungsform wie bereits oben beschrieben, bei jenen Enzymen, die ein divalentes Ion benötigen, ein divalentes Ion vor. Bevorzugt sind Mg²⁺, Mn²⁺.

Bevorzugte Kombinationen von Enzymen sind HIV reverse Transkriptase oder M-MLV reverse Transkriptase oder EAIV reverse Transkriptase oder AMV reverse Transkriptase oder *Thermus thermophilus* DNA Polymerase I oder M-MLV RNAse H, Superscript, Superscript II, Superscript III oder Monstersript (Epicentre) oder Omniscript Reverse Transkriptase (Qiagen) oder Sensiscript Reverse Transkriptase (Qiagen), ThermoScript, Thermo-X (beide Invitrogen) oder eine Mischung von zwei oder mehr Enzymen mit reverser Transkriptaseaktivität und Poly-(A)-Polymerase aus *Escherichia coli.* Außerdem HIV reverse Transkriptase oder M-MLV reverse Transkriptase oder EAIV reverse Transkriptase oder AMV reverse Transkriptase oder *Thermus thermophilus* DNA Polymerase I oder M-MLV RNAse H, Superscript, Superscript II, Superscript III oder Monstersript (Epicentre) oder Omniscript Reverse Transkriptase (Qiagen) oder Sensiscript Reverse Transkriptase (Qiagen), ThermoScript, Thermo-X (beide Invitrogen) oder eine Mischung von zwei oder mehr Enzymen mit reverser Transkriptaseaktivität und Poly-(A)-Polymerase aus Hefe.

Dem Fachmann ist bekannt, dass hohe Temperaturen bei der reversen Transkrigtion dazu führen, dass Probleme mit Sekundärstrukturen keine so entscheidende Rolle spielen. Zudem führen hohe Temperaturen bei bestimmten Enzymen dazu, dass die Spezifität der reversen Transkription dadurch steigt, dass Fehlpaarungen und falsches Priming unterbunden werden. Somit wird in einer Ausführungsform der vorliegenden Erfindung eine reverse Transkriptase verwendet, die thermophil ist. Bevorzugt wird ein Enzym welches eine optimale Nukleinsäuresyntheseaktivität bei zwischen 45°C und 85°C aufweist, bevorzugter zwischen 55°C und 80°C am bevorzugtesten zwischen 60°C und 75°C. Bevorzugt wird *Thermus thermnophilus* (Tth) DNA Polymerase I.

Sofern es sich bei dem Enzym mit Polyadenylierungsaktivität um ein nicht-thermophiles Enzym handelt und bei dem Enzym mit reverser Transkriptaseaktivität um ein thermophiles Enzym handelt, kann erfindungsgemäß das Verfahren in mehreren Temperaturschritten erfolgen, wobei der erste Temperaturschritt eine Temperatur zur Anwendung kommen lässt, die die optimal Temperatur für das Enzym mit Polyadenylierungsaktivität ist, und der zweite Temperaturschritt eine Temperatur zur Anwendung kommen lässt, die die optimale Temperatur für das Enzym mit reverser Transkriptaseaktivität ist.

Wird beispielsweise die AMV-reverse Transkriptase verwendet, so findet der zweite Temperaturschritt bei 42°C statt, wohingegen der erste Temperaturschritt, welche primär der Aktivität des Enzyms mit Polyadenylierungsaktivität zukommt, bei einer Temperatur von 37°C statt. Es ist allerdings auch die Durchführung bei einer konstanten Temperatur möglich.

Der Fachmann wird in der Lage sein, die Temperaturen so zu wählen, dass die jeweiligen Enzymaktivitäten zum tragen kommen. Wird beispielsweise eine Kombination aus Poly-(A)-Polymerase aus *Escherichia coli* einhergehend mit DNA-Polymerase aus *Thermus thermophilus* verwendet, so sieht der Ablauf der Temperaturen wie folgt aus: Zunächst wird bei 37°C inkubiert und anschließend wird bei 55 bis 70°C inkubiert. Erfindungsgemäß kann also ein nicht thermostabiles Enzym mit einem thermostabilen Enzym kombiniert werden. In diesem Fall richten sich die Temperaturschritte danach, welches der beiden Enzyme Polyadenylierungsaktivität aufweist. Erfindungsgemäß ist es bevorzugt, dass das Enzym mit reverser Transkriptaseaktivität thermostabil ist. Im umgekehrten Fall, und dies leuchtet dem Fachmann ein, kann es sein, dass durch die Inkubation bei hoher Temperatur im Polyadenylierungsschritt, das Enzym mit reverser Transkriptaseaktivität in Teilen oder ganz inaktiviert wird. Somit ist auch bevorzugt, wenn beide Enzyme thermostabil sind.

Weiterhin ist dem Fachmann bekannt, dass die Enzyme sehr unterschiedlich prozessiv sind, so dass der Fachmann Enzyme mit unterschiedlicher Prozessivität so kombinieren kann, dass Templates mit unterschiedlicher Länge unterschiedlich gut in cDNA umgewandelt werden. Durch Verwendung entsprechender Mengen der jeweiligen Enzyme, einer oder mehrerer geeigneter Inkubationstemperaturen und Inkubationszeiten ist es dem Fachmann möglich zufriedenstellende Ergebnisse zu erzielen.

Das erfindungsgemäße Verfahren umfasst vorzugsweise zusätzlich Poly-(C)-Polynucleotide. Das erfindungsgemäße Verfahren umfasst besonders vorzugsweise zusätzlich Poly-(C)-Polyribonucleotide. Vorzugsweise werden 1 ng bis 300 ng Poly-(C)-Polyribonucleotide pro 20 µl eingesetzt, vorzugsweise 10 ng bis 150 ng Poly-(C)-Polyribonucleotide pro 20 µl Reaktion eingesetzt, besonders bevorzugt werden 25 ng bis 100 ng Poly-(C)-Polyribonucleotide pro Reaktion eingesetzt und am Bevorzugtesten 50 ng bis 75 ng Poly-(C)-Polyribonucleotide pro 20 µl Reaktion eingesetzt.

Die erfindungsgemäße Reaktion kann weiter Reagenzien wie beispielsweise Volume Excluder, einen Singel-strand Binding Protein, DTT und/oder Kompetitomukleinsäuren umfassen.

Wird ein Volume Excluder eingesetzt, so ist dieser ausgewählt aus der Gruppe umfassend Dextran, Polyethylenglycol, und in EP1411133A1 werden erfindungsgemäße Volume Excluder genannt.

In einer bevorzugten Ausführungsform handelt es sich bei der Kompetitor-Nukleinsäure um eine homopolymere Ribonukleinsäure am bevorzugtesten um Polyadenoribonukleinsäure. Beispiele werden in US 6,300,069 offenbart.

Das erfindungsgemäße Verfahren umfasst vorzugsweise zusätzlich Poly-(C)-Polynucleotide. Das erfindungsgemäße Verfahren umfasst besonders vorzugsweise zusätzlich Poly-(C)-Polyribonucleotide. Vorzugsweise werden 1 ng bis 300 ng Poly-(C)-Polyribonucleotide pro 20 µl eingesetzt, vorzugsweise 10 ng bis 150 ng Poly-(C)-Polyribonucleotide pro 20 µl Reaktion eingesetzt, besonders bevorzugt werden 25 ng bis 100 ng Poly-(C)-Polyribonucleotide pro Reaktion eingesetzt und am Bevorzugtesten 50 ng bis 75 ng Poly-(C)-Polyribonucleotide pro 20 µl Reaktion eingesetzt.

Es ist für den Fachmann offensichtlich, dass es von Vorteil sein kann zu verhindern, dass die Kompetitor-Nukleinsäure selbst als Substrat für die Poly-(A)-Polymeraseaktivität dient. Eine mögliche Lösung ist die Blockierung der 3' OH Gruppe der Kompetitor-Nukleinsäure. Entsprechende Lösungen wie z.B. Verwendung eines 3' Phosphat, Einbau eines Didesoxynukleotids oder inverser Basen sind dem Fachmann bekannt.

Es ist für den Fachmann ebenfalls offensichtlich, dass es von Vorteil sein kann zu verhindern, dass die Kompetitor-Nukleinsäure selbst als Substrat für die Reverse Transkriptaseaktivität dient. Dies kann durch Auswahl einer Kompetitor-Nukleinsäure sichergestellt werden, welche nicht unter den gegebenen Reaktionsbedingungen in cDNA umgeschrieben werden kann, z.B. weil die verwendeten Primer nicht an diese hybridisieren können. Eine weitere mögliche Lösung ist die Blockierung der 3' OH Gruppe der Kompetitor-Nukleinsäure. Entsprechende Lösungen wie z.B. Verwendung eines 3' Phosphat, Einbau eines Didesoxynukleotids oder inverser Basen sind dem Fachmann bekannt.

Die Erfindung betrifft weiterhin ein Reaktionsgemisch umfassend ein erstes Enzym mit Polyadenylierungsaktivität, ein zweites Enzym mit reverser Transkriptaseaktivität, Kompetitor Poly-(C)-Polynukleotide, optional einen Puffer, optional mindestens ein Ribonukleotid, optional mindestens ein Desoxyribonukleotid und optional ein Anker Oligonukleotid. Bevorzugt umfasst das Anker Oligonukleotid einen homopolymer Anteil, eine Ankersequenz und/oder einen Tail. Bevorzugt umfasst das Reaktionsgemisch zusätzlich random Primer. Die zusätzlich Verwendung von random Primern hat den Vorteil, dass auch 5' Enden von langen Transkripten effizient umgeschrieben werden, was bei quantitativen Analysen wichtig ist. Das Reaktionsgemisch kann die gleichen Agenzien beinhalten wie sie für das erfindungsgemäße Verfahren zur Anwendung kommen.

In einer Ausführungsform der Erfindung handelt es sich bei dem Anker Oligonukleotid um ein homopolymeres Oligonukleotid, welches ausgewählt ist aus der Gruppe umfassend, ein Poly-(A)-Oligonukleotid, Poly-(C)-Oligonukleotid, Poly-(T)-Oligonukleotid, Poly-(G)-Oligonukleotid. Poly-(U)-Oligonukleotid, Poly-(A)-Oligonukleotid zusätzlich umfassend einen 5'-Tail, Poly-(C)-Oligonukleotid zusätzlich umfassend einen 5'-Tail, Poly-(T)-Oligonukleotid zusätzlich umfassend einen 5'-Tail, Poly-(G)-Oligonukleotid zusätzlich umfassend einen 5'-Tail und Poly-(U)-Oligonukleotid zusätzlich umfassend einen 5'-Tail. Bevorzugt wird ein Poly-(T)-Oligonukleotid, welches optional wie bereits oben ausgeführt, zusätzlich einen 5'-Tail haben kann.

Das erfindungsgemäße Anker Oligonukleotid ist in der Regel zwischen 6 und 75 Nukleotide lang. Es kann jedoch bis zu ca. 150 Nukleotide lang sein. Ist das Anker Oligonukleotid synthetisch, so ergibt sich die maximale Länge aus den technischen Limitationen der DNA Synthese. Optional umfasst das Anker Oligonukleotid einen 5'-Tail und/oder eine Ankersequenz. Ein 5'-Tail ist eine zusätzliche Nukleotidsequenz am 5'-Ende des Oligonukleotids, die beispielsweise dazu dient eine Klonierungssequenz, Primer- und/oder Sonden-Bindungsstellen oder eine beliebige andere Sequenz einzuführen. Das identifizieren von geeigneten Sequenzen für den 5' Tail ist für den Fachmann basierend auf den Anforderungen der jeweilige Anwendung möglich.

Am 3'-Ende des Anker Oligonukleotids kann eine zusätzliche Ankersequenz typischerweise mit einer Länge von ein bis fünf weitere Nukleotiden enthalten sein. Die Ankersequenz kann eine Länge von mindestens einer Base aufweisen, wobei die erste Position in einer bevorzugten Ausführungsform eine degenerierte Base ist, die alle Basen außer der im homopolymer Anteil des Anker Oligonukleotids verwendeten Base enthält. Hierauf können weitere Basen folgen. Diese können auch degeneriert sein. In einer bevorzugten Ausführungsform ist hier die Verwendung von N. Wobbles sinnvoll, wobei N= A, C, G, T oder entsprechende Analoga.

Der optionale 5'-Tail umfasst zusätzliche 1-100 Nukleotide, welche für nachfolgende Analysen Verwendung finden können. So kann in einer bevorzugten Ausführungsform der 5'Tail die Bindungssequenz für ein Oligonukleotid wie z.B. eine oder mehrere DNA Sonden und/oder einen oder mehrere PCR Primer enthalten. Die verwendeten Sequenzen für den 5'Tail werden bevorzugt so ausgewählt, dass diese mit dem erfindungsgemäßen Verfahren kompatibel sind. Dies umfasst z.B. die Auswahl solcher Sequenzen, die keine ungewünschten Nebenreaktionen, sowohl im erfindungsgemäßen Verfahren als auch bei nachfolgen Analyseverfahren, hervorrufen.

Das erfindungsgemäße Reaktionsgemisch umfasst das erfindungsgemäße Anker Oligonukleotid, welches eine Länge zwischen 10 und 150 Nukleotiden hat, und optional am 3'-Ende eine eins bis fünf Nukleotide lange erfindungsgemäße Ankersequenz trägt. Das erfindungsgemäße Reaktionsgemisch umfasst das Anker Oligonukleotid, welches wie oben beschrieben zum Beispiel eine Desoxyribonukleinsäure (DNA) ist, eine Peptidnukleinsäure (PNA) ist oder eine locked-Nukleinsäure (LNA) ist. Das erfindungsgemäße Reaktionsgemisch umfasst in einer bevorzugten Ausführungsform ein erfindungsgemäßes Anker Oligonukleotid, welches ein Poly-(T)-Oligonukleotid ist und zusätzlich einen 5'-Tail trägt, wobei es sich bei dem Oligonukleotid um eine Desoxyribonukleinsäure handelt, die 10 bis 75 Nukleotide lang ist und als Gemisch vorliegt, wobei am 3'-Ende eine Ankersequenz vorhanden ist, bestehend aus jeweils einem Nukleotid, welches ausgewählt ist aus der Gruppe umfassend A, G und C, optional gefolgt von ein bis fünf weiteren Nukleotiden umfassend alle vier Basen A, C, G, und T oder entsprechende Analoga.

Erfindungsgemäße Anker Oligonukleotide des Reaktionsgemisches sind in Fig. 12 gezeigt.

Das erfindungsgemäße Reaktionsgemisch umfasst weiter mindestens ein Ribonukleotid wie sie oben für das erfindungsgemäße Verfahren beschrieben wurden. Insbesondere umfasst das erfindungsgemäße Reaktionsgemisch mindestens ein Ribonukleotid ausgewählt aus ATP, TTP, CTP, GTP, UTP oder entsprechenden Basenanaloga. Die Ribonukleotide können optional, wie oben beschrieben, modifiziert oder markiert sein. Das erfindungsgemäße Reaktionsgemisch umfasst Desoxyribonukleotide wie es für das erfindungsgemäße Verfahren beschrieben wurde. Insbesondere umfasst das erfindungsgemäße Reaktionsgemisch eines oder mehrere Desoxyribonukleotide wie beispielsweise dATP, dCTP, dGTP, dUTP und/oder dTTP. In einer bevorzugten Ausführungsform wird eine Mischung von Desoxyribonukleotiden verwendet, welche eine cDNA Synthese erlauben. Diese Desoxyribonukleotide können optional modifiziert oder markiert sein.

Sofern ein Desoxyribonukleotid des erfindungsgemäßen Reaktionsgemisches markiert ist, kann die Markierung ausgewählt werden aus der Gruppe umfassend ³²P, ³³P, ³⁵S, ³H, ein fluoreszierender Farbstoff wie beispielsweise Fluoresceinisothiocyanate (FITC), 6-Carboxyfluorescein (FAM), Xanthen, Rhodamine, ,6-Carboxy-2',4',7',4,7-hexachlorofluorescein (HEX), 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine 110; Cy3, Cy5, Cy7, Coumarine, wie Umbelliferone, Benzimide, wie Hoechst 33258; Phenanthridine, wie Texas Red, Ethidiumbromide, Acridinfarbstoffe, Carbazolfarbstoffe, Phenoxazinefarbstoffe, Porphyrinfarbstoffe, Polymethinfarbstoffe, Cyaninfarbstoffe, wie Cy3, Cy5, BODIPY-Farbstoffe, Quinolinfarbstoffe und Alexa Farbstoffe. Andere Markierungen wie das Einfügen von Biotin oder einem oder mehreren Haptenen wie z.B. Digoxigenin, welchen einen direkten oder indirekten Nachweis der Nukleinsäure erlauben. Indirekte Nachweise wie z.B. über Antikörper, welche wiederum einen z.B. enzymatischen Nachweis über ein am Antikörper gekoppeltes Enzym. Auch über das Einbringen von Nanopartikeln, die z.B. an Antikörper oder einen Affinitätsliganden gekoppelt sind, ist ein indirekter Nachweis möglich.

Das erfindungsgemäße Reaktionsgemisch umfasst jeweils ein Desoxyribonukleotid bei einer Konzentration von 0,01 mM bis 10 mM. Bevorzugt liegt das einzelne Desoxyribonukleotid A, C, G und T in einer Konzentration von jeweils 0,2 mM bis 2 mM vor. Besonders bevorzugt ist es, wenn die Desoxyribonukleotide A, C, G und T gemeinsam vorliegen. Jedes einzelne liegt in dieser bevorzugten Ausführungsform in einer Konzentration von 0,5 mM vor.

Das erfindungsgemäße Reaktionsgemisch umfasst weiterhin einen Puffer. Dieser Puffer hat einen pH von 6 bis 10. Im erfindungsgemäßen Reaktionsgemisch befinden sich weiterhin Mg²⁺ - Ionen. In einer besonders bevorzugten Ausführungsform hat das erfindungsgemäße Reaktionsgemisch einen Puffer mit einem pH von 6,8 bis 9. Das Reaktionsgemisch kann weiter zusätzlich Ionen umfassen, die ausgewählt sein können aus der Gruppe umfassend Mn²⁺, K⁺, NH⁴⁺ und Na⁺. Wesentlich für das erfindungsgemäße Reaktionsgemisch ist das Vorliegen zwei unterschiedlicher Enzymaktivitäten. Das erfindungsgemäße Reaktionsgemisch umfasst wenigstens eine erste Enzymaktivität mit Polyadenylierungsaktivität sowie zweitens eine zweite Enzymaktivität mit reverser Transkriptaseaktivität. Die bevorzugten Ausführungsformen dieser Aktivitäten wurden bereits für das Verfahren oben beschrieben. Das Reaktionsgemisch kann wie das Verfahren oben weiterhin zusätzliche Substanzen, wie beispielsweise einen Volume Excluder, einen Single-strand Binding Protein, DTT oder eine oder mehrere Kompetitor-Nukleinsäuren umfassen.

Wird ein Volume Excluder eingesetzt, so ist es bevorzugt, dass dieser ausgewählt ist aus der Gruppe umfassend Dextran, Polyethylenglycol. Weitere Volume Excluder gemäß der vorliegenden Erfindung finden sich in EP1411133A1.

Umfasst das Reaktionsgemisch optional eine Kompetitor-Nukleinsäure so ist diese ausgewählt aus der Gruppe umfassend homopolymere Ribonukleinsäuren und Polyadenoribonukleinsäure. Weitere erfindungsgemäße Kompetitor-Nukleinsäuren sind in US 6,300,069 offenbart.

Das erfindungsgemäße Reaktionsgemisch umfasst Kompetitor Poly-(C)-Polynucleotide. Das erfindungsgemäße Reaktionsgemisch umfasst besonders vorzugsweise zusätzlich Poly-(C)-Polyribonucleotide. Vorzugsweise werden 1 ng bis 300 ng Poly-(C)-Polyribonucleotide pro 20 µl eingesetzt, vorzugsweise 10 ng bis 150 ng Poly-(C)-Polyribonucleotide pro 20 µl Reaktion eingesetzt, besonders bevorzugt werden 25 ng bis 100 ng Poly-(C)-Polyribonucleotide pro Reaktion eingesetzt und am Bevorzugtesten 50 ng bis 75 ng Poly-(C)-Polyribonucleotide pro 20 µl Reaktion eingesetzt.

Die Erfindung betrifft weiterhin einen Kit, umfassend ein Reaktionsgemisch, wie er oben beschrieben wurde. Das Reaktionsgemisch liegt in einer bevorzugten Ausführungsform in einem einzigen Reaktionsgefäß vor. In einer anderen Ausführungsform umfasst der Kit ein Reaktionsgefäß, umfassend das Enzym mit Polyadenylierungsaktivität, das Enzym mit reverser Transkriptaseaktivität, optional die Desoxyribonukleotide, optional mindestens ein Ribonukleotid, optional einen Puffer enthaltend Mg²⁺ und optional ein oder mehr Oligodesoxyribonukleotide im Sinne der Erfindung. Optional kann das Reaktionsgefäß in dem erfindungsgemäßen Kit weitere Bestandteile enthalten, wie sie für das erfindungsgemäße Reaktionsgemisch angegeben wurden. Der Kit kann weiterhin eine Sonde für den 5' Tail des erfindungsgemäßen Anker Oligonukleotids umfassen. Außerdem kann das Kit einen oder mehrere weitere Desoxyribonukleotide enthalten, so z.B. einen generischen Primer zum Nachweis der durch die Reverse Transkription eingebrachten Tail Sequenz. Das Reaktionsgemisch kann in "Pellet-Form" vorliegen also z. B. lyophilisiert. Dem Fachmann sind weitere Verfahren der Bereitstellung bekannt, die z.B. nicht flüssige Formen beinhalten.

Weiterhin kann der Kit optional kombiniert sein mit Reagenzien wie sie für die PCR-Reaktion oder Real-time PCR Reaktion notwendig sind. Bevorzugt sind dies Reagenzien für mindestens eine PCR Reaktion, die den Nachweis mindestens einer der im Erfingsgemäßen Verfahren generierten cDNA erlaubt.

Der Kit kann weiterhin optional Random-Primer umfassen sowie optional ein oder mehrere Primer oder Primer/Sonden zum Nachweis weiterer Target-Gene in singleplex oder multiplex PCR-Reaktionen und/oder Real-time singleplex oder multiplex PCR Reaktionen.

Das Reaktionsgemisch, das erfindungsgemäße Verfahren bzw. der Kit, können weiter Targetspezifische Primer enthalten. Die Länge der Target-spezifischen Primer sollte so gewählt werden, dass ein spezifischer Nachweis in einer PCR-Reaktion möglich ist, die Sequenz des Targetprimers sollte so spezifisch sein, dass eine Bindung an nur eine Stelle in der generierten cDNA-Sequenz möglich ist. In aller Regel hat ein solcher Primer eine Länge von 15 bis 30 Nukleotiden, bevorzugt von 17 bis 25 Nukleotiden.

In einer besonders bevorzugten Ausführungsform umfasst das Reaktionsgemisch das Enzym mit Polyadenylierungsaktivität und das Enzyme mit reverser Transkriptaseaktivität als Zweienzyme Pre-mix. Das Kit umfasst in dieser besonders bevorzugten Ausführungsform das Zwei-enzympre-mix-reaktionsgemisch in einem Reaktionsgefäß und in einem getrennten Gesäß einen Puffer, Mg²⁺, rNTP(s), dNTP(s), optional ein erfindungsgemäßes Anker Oligonukleotid und optional random Primer sowie weiterhin optional ein volume excluding Reagenz und/oder eine Kompetitor-Nukleinsäure.

Das erfindungsgemäße Verfahren kann, wie bereits oben ausgeführt, in einem oder mehreren Temperaturschritten stattfinden. In einer bevorzugten Ausführungsform findet das erfindungsgemäße Verfahren bei einem einzigen Temperaturschritt für die Inkubation statt und einem weiteren Temperaturschritt für die Inaktivierung für die Enzyme statt. So ist in einer bevorzugten Ausführungsform der Inkubationsschritt bei der die Enzyme Aktivität entfalten, etwa 37°C. Die Inkubationszeit beträgt etwa, 1 bis 120 Minuten, bevorzugt 5 bis 90 Minuten, bevorzugter 10 bis 75 Minuten, noch bevorzugter 15 bis 60 Minuten, noch bevorzugter 20 bis 60 Minuten am bevorzugtesten 50 bis 70 Minuten. Eine darüber hinausgehende Inkubationszeit ist in aller Regel nicht schädlich. In einem weiteren Schritt, welcher der Denaturierung der Enzyme dient, wird eine Temperatur von wenigstens 65°C höchstens jedoch 100°C verwendet. Bevorzugterweise wird eine Temperatur von etwa 80-95°C verwendet. Die Denaturierung geschieht für einen Zeitraum von wenigstens 1 Minute höchstens jedoch für 30 Minuten. In einer bevorzugten Ausführungsform wird für einen Zeitraum von 5 Minuten denaturiert.

Das erfindungsgemäße Verfahren zur Generierung von cDNA kann im Anschluss eine Polymerasekettenreaktion umfassen. Ist dies der Fall, so wird bevorzugterweise ein für den während cDNA Synthese eingebrachten Tail spezifischer Primer und/oder ein spezifischer Primer dem erfindungsgemäßen Reaktionsgemisch zugegeben. Das Reaktionsgemisch enthält dann auch weiterhin eine thermostabile DNA-Polymerase.

Die im Anschluss stattfindende PCR-Reaktion kann auch eine quantitative PCR-Reaktion sein. Sie kann auf einem Array stattfinden, in einem mikrofluidischen System stattfinden, in einer Kapillare stattfinden oder aber eine real-time PCR sein. Andere Varianten der PCR sind dem Fachmann bekannt und sind gleichermaßen vom erfindungsgemäßen Verfahren umfasst.

Ebenfalls hierin beschrieben ist ein Verfahren zur reversen Transkription von RNA in DNA, wobei das Verfahren folgende Schritte umfasst, Bereitstellung einer Probe umfassend RNA, Zugabe eines ersten Enzyms mit reverser Transkriptaseaktivität, eines Puffers, mindestens eines Desoxyribonukleotids, eines Oligonukleotids, Inkubation der Agenzien bei einem oder mehreren Temperaturschritten, welche so gewählt sind, dass das Enzym Aktivität zeigt, wobei die Reaktion zusätzlich Poly-(C)-Polynukleotide umfasst.

Vorzugsweise ist das Enzyms mit reverser Transkriptaseaktivität HIV reverse Transkriptase, M-MLV reverse Transkriptase, EAIV reverse Transkriptase, AMV reverse Transkriptase, *Thermus thermophilus* DNA Polymerase I, M-MLV RNAse H⁻⁽Superscript, Superscript II, Superscript III), Monstersript (Epicentre), Omniscript Reverse Transkriptase (Qiagen), Sensiscript Reverse Transkriptase (Qiagen), ThermoScript, Thermo-X (beide Invitrogen) oder eine Mischung von zwei oder mehr Enzymen mit reverser Transkriptaseaktivität und Poly-(A)-Polymerase aus *Escherichia coli.* Besonders bevorzugt ist HIV reverse Transkriptase.

Vorzugsweise umfasst die Reaktion Poly-(C)-Polyribonucleotide. Vorzugsweise werden 1 ng bis 300 ng Poly-(C)-Polyribonucleotide pro 20 µl eingesetzt, vorzugsweise 10 ng bis 150 ng Poly-(C)-Polyribonucleotide pro 20 µl Reaktion eingesetzt, besonders bevorzugt werden 25 ng bis 100 ng Poly-(C)-Polyribonucleotide pro Reaktion eingesetzt und am Bevorzugtesten 50 ng bis 75 ng Poly-(C)-Polyribonucleotide pro 20 µl Reaktion eingesetzt.

In einer bevorzugten Ausführungsform handelt es sich bei der Probe um eine Ribonukleinsäure, die ausgewählt ist aus der Gruppe umfassend prokaryontische Ribonukleinsäuren, eukaryontische Ribonukleinsäuren, virale Ribonukleinsäuren, Ribonukleinsäuren deren Ursprung ein Archae-Organismus ist, microRibonukleinsäuren (miRNA), small nucleolar Ribonukleinsäuren (snoRNA), messenger Ribonukleinsäure (mRNA), Transfer-Ribonukleinsäuren (tRNA), nicht-polyadenylierte Ribonukleinsäuren im allgemeinen, sowie ribosomale Ribonukleinsäuren (rRNA) Darüber hinaus eine Mischung von zwei oder mehr der genannten Ribonukleinsäuren In der Probe kann natürlich auch bereits poly-A RNA enthalten sein.

Als template kann eine RNA dienen die ausgewählt ist aus der Gruppe umfassend, eukaryontische Ribonukleinsäuren, mRNA, prokaryontische Ribonukleinsäuren, miRNA, snoRNA und rRNA. In der bevorzugtesten Ausführungsform der vorliegenden Erfindung umfasst die Probe eine Ribonukleinsäure, die ausgewählt ist aus der Gruppe umfassend miRNA und snoRNA. Bevorzugt werden weiter Mischproben aus unterschiedlichen Mengen von Ribonukleinsäuren unterschiedlicher Art einhergehend mit anderen Stoffen.

Aufgrund der vorliegenden Vorteile des erfindungsgemäßen Verfahrens konnten die Erfinder zeigen, dass es möglich ist, miRNAs effizient und ohne Kontamination bereitzustellen und zu charakterisieren. Kleine RNA Mengen allgemein lassen sich mit dem Verfahren gut Reverstranskribieren.

Die Beschreibung betrifft ferner einen Kit für die Reverse Transkription umfassend, ein Enzym mit Reverser Transkriptaseaktivität und Poly-(C)-Polynukleotide bevorzugt, Poly-(C)-Polyribonukleotide.

In einer Ausführungsform wird die RNA zunächst Polyadenyliert bevor die Probe reverse transkribiert wird.

### BEISPIELE

### Beispiel 1:

Demonstration der Machbarkeit eines gekoppelten einstufigen Prozesses von Poly-A-Reaktion und reverser Transkription im gleichen Reaktionsgefäß; Effekt verschiedener Puffer auf die Effizienz des Nachweises eines 22-mer RNA-Oligonukleotids

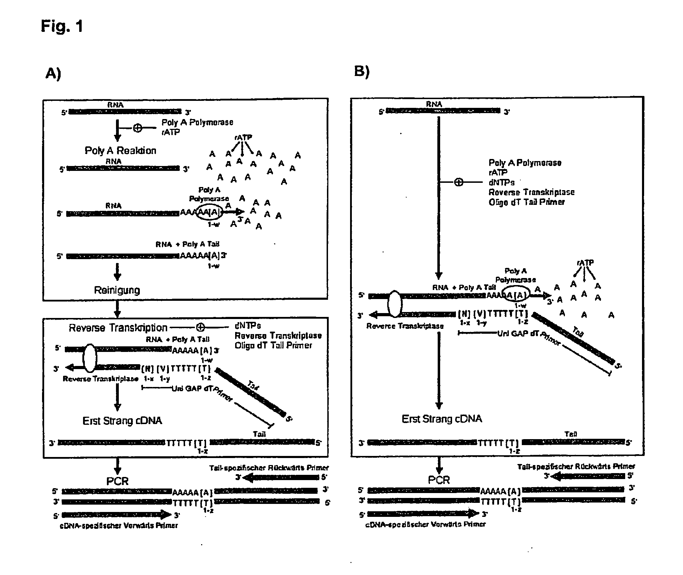

In diesem Experiment sollte die Machbarkeit eines gekoppelten einstufigen Prozesses von Poly-(A)-Polymerase Reaktion und reverser Transkription im gleichen Reaktionsgefäß demonstriert werden. Zu diesem Zweck wurde der gekoppelte einstufige Prozess unter verschiedenen Bedingungen durchgeführt. Dies waren zum einen der mit der Poly-(A)-Polymerase gelieferte Puffer, zum anderen der mit der reversen Transkriptase gelieferte Puffer. Weiterhin wurde eine Mischung von Puly-(A)-Polymerase- und reversen Transkriptase-Puffern getestet. Als Kontrolle wurde die Reaktion in einem zweistufigen Verfahren in Anlehnung an die Abbildung 1 A durchgeführt.

Die Reaktionen wurden wie in Tabelle 1 angegeben zusammengesetzt.

**Tabelle 1: Poly-A-Reaktion und Reverse Transkription**

| | | | Ansatz 3 a/b | Ansatz 4 a/b | |
|---|---|---|---|---|---|
| | Ansatz 1 a/b | Ansatz 2 a/b | | | |
| | Angaben der finalen Konzentrationen | | | zweistufiges Verfahren | |
| Reagenzien | PAP Puffer | RT Puffer | Mischung beider Puffer | 1.) (PAP Reaktion | 2.) RT Reaktion |
| 5x PAP Puffer | 1 x | | 1 x | 1 x | |
| 10x RT Puffer | | 1 x | 1 x | | 1x |
| MnCl₂, Lösung 25 mM | 2,5 mM | | 2,5 mM | 2,5 mM | |
| rATP, 10 mM | 1mM | 1 mM | 1 mM | 1 mM | |
| Poly-(A)-Polymerase 2U/ µl | 4U (0,2U/µl) | 2U (0,1U/µl) | 4U (0,2U/µl) | 2U (0,2U/µl) | |
| dNTP Mix ( dA, dT, dG, dC je 5mM) | 0,5 mM | 0,5 mM | 0,5 mM | | 0,5 mM |
| UniGAPdT Primer 10 µM | 1 µM | 1 µM | 1 µM | | 1 µM |
| 1 RNase Inhibitor 10 U/µl | 10 U | 10 U | 10 U | | 10 U |
| Sensiscript Reverse Transkriptase ' | 1 µl | 1 µl | 1 µl | | 1 µl |
| RNase freies Wasser | variabel | variabel | variabel | variabel | variabel |
| a) mleu7a | 2x10E9 Kopien | 2x10E9 Kopien | 2x10E9 Kopien | 2x10E9 Kopien | 10 µl PAP Reaktion 1.) |
| b) neg Kontrolle (H₂O anstatt mleu7a) | H₂O | H₂O | H₂O | H₂O | |
| a) Mais RNA | 50 ng | 50 ng | 50 ng | 50 ng | |
| b) neg Kontrolle (H₂O anstatt Mais RNA) | H₂O | H₂O | H₂O | H₂O | |
| Gesamtvolumen | 20 µl | 20 µl | 20 µl | 10 µl | 20 µl |
| | | | | | |
| Inkubation | 1 Std. 37°C 5 min 93°C | | | 1 Std 37°C, dann weiter bei 2.) RT Reaktion | 1 Std. 37°C 5 min 93°C |

| | | | | | |
|---|---|---|---|---|---|
| PAP: Poly A Polymerase RT: Reverse Transkription rATP: Adenosine 5'-Triphosphate | | | | | |

Dazu wurden die in Tabelle 2 angegebenen Reagenzien verwendet.

**Tabelle 2: Materialien für die Poly-A-Reaktion und Reverse Transkription**

| | |
|---|---|
| Poly-(A)-Polymerase | Ambion; Material Nummer 80U: 2030 |
| Sensiscript RT Kit | Qiagen; Material Nummer 50rxn: 205211 |
| Adenosine 5'-Triphosphate (rATP) | Amersham; Material Nummer 25µmol: 27-2056-01 |
| RNase Inhibitor | Promega: Material Nummer: N2511 |
| Uni GAP dT Primer | |
| Mais RNA | Aus 1g gemörserten Maisblättern mit Qiagen RNeasy Mini Kit (Cat. No. 74106); Plant Protokoll mit 10 fachem upscale (Maxi shredder und Säule) |
| mlcu7a RNA Oligonukleotid | 5'-UGA GGU AGU AGG UUG UAU AGU U-3' |

Es wurden jeweils Reaktionen mit Template (1a, 2a, 3a, 4a, alle mit synthetischem RNA Oligonukleotid in einem Hintergrund von Mais RNA) bzw. ohne Template (1b, 2b, 3b, 4b, alle wurden H₂O anstatt Template zugesetzt) durchgeführt. Die Reaktionen ohne Template wurden als Kontrolle für ein mögliches Auftreten von unspezifischen Hintergrund durchgeführt. Als Hintergrund RNA wurde Mais RNA gewählt, da die nachzuweisende Sequenz des 22-mer RNA Oligonukleotids bei Mais nicht vorkommt.

Nach der Inaktivierung der Enzyme (siehe Tabelle: 5 min bei 93°C) wurden je 2 µl der Ansätze 1 a/b bis 4 a/b als Template in eine real-time PCR eingesetzt. Der Ansatz der real-time PCR erfolgte in Dreifachansätzen wie in Tabelle 3 angegeben mit QuantiTect SYBR Green PCR Kit (Katalog Nr. 204143) und den in Tabelle 4 angegebenen Primern.

**Tabelle 3: Komponenten für SYBR Green Real-time PCR**

| | Finale Konzentration |
|---|---|
| 2x SYBR Green PCR Master Mix | 1x |
| Hum Uni Primer 10 µM | 0,5 µM |
| miRNA Primer let7short 10µM | 0,5 µM |
| RNase-free water | variabel |
| PAP-/ RT- Reaktion | 2 µl |
| Finales Reaktionsvolumen | 20 µl |

**Tabelle 4: Materialien für die SYBR Green PCR**

| QuantiTect SYBR Green PCR Kit (200) | Qiagen; Material Nummer: 204143 |
|---|---|
| | |
| Let 7short (spezifischer miRNA Primer) | 5'-GAG GTA GTA GGT TGT ATA G-3' |
| Hum Uni Primer | 5'-AAC GAG ACG ACG ACA GAC-3' |

Die im universellen Tail-Primer Hum Uni Primer enthaltene Sequenz 5'-AAC GAG ACG ACG ACA GAC-3' wurde in US2003/0186288A1 beschrieben.

Das PCR Protokoll bestand aus einer initialen Reaktivierung der im QuantiTect SYBR Green PCR Master Mix enthaltenen HotStarTaq Polymerase für 15 min bei 95°C, gefolgt von 40 Zyklen mit 15 sec 94°C, 30 sec bei 52°C und 30 sec bei 72°C (siehe Tabelle 5).

**Tabelle 5: PCR Protokoll für SYBR Green Real-time PCR**

| | | |
|---|---|---|
| PCR Initiale Reaktivierung | 15 min 95°C | |
| Denaturierung | 15 sec 94°C | 40x |
| Annealing | 30 sec 52°C | |
| Extension (Datenaufnahme) | 30 sec 72°C | |
| Schmelzkurve | | |

Die Aufnahme der Fluoreszenzdaten erfolgte während des 72°C Extensions-Schrittes. Die PCR-Analysen wurden mit einem ABI PRISM 7700 (Applied Biosystems) in einem Reaktionsvolumen von 20 µl durchgeführt.

Die PCR Produkte wurden anschließend einer Schmelzkurvenanalyse unterzogen. Diese wurde auf einem ABI PRISM 7000 Real-time PCR Instrument durchgeführt.

**Tabelle 6: Ergebnisse einer Real-time PCR Analyse der Ansätze aus Tabelle 1**

| Detector | PAP/ RT | Template | Ct | Ct Mittel | CV in % |
|---|---|---|---|---|---|
| SYBR Green | 1) PAP Puffer | a) mleu7a 2x10^8 Kopien + Mais cDNA 5 ng | 25,64 | | |
| | | | 25,22 | 25,50 | 0,95 |
| | | | 25,64 | | |
| | | b) H₂O in PAP - RT Reaktion | Kein Ct | | |
| | | | Kein Ct | Kein Ct | |
| | | | Kein Ct | | |
| | 2) RT Puffer | a) mleu7ä 2x10^8 Kopien + Mais cDNA 5 ng | 17,43 | | |
| | | | 17,31 | 17,32 | 0,58 |
| | | | 17,23 | | |
| | | b) H₂O in PAP - RT Reaktion | Kein Ct | | |
| | | | Kein Ct | Kein Ct | |
| | | | Kein Ct | | |
| | 3) Mischung beider Puffer | a) mleu7a 2x10^8 Kopien + Mais cDNA 5 ng | 30,41 | | |
| | | | 30,23 | 30,28 | 0,36 |
| | | | 30,21 | | |
| | | b) H₂O in PAP - RT Reaktion | Kein Ct | | |
| | | | Kein Ct | Kein Ct | |
| | | | Kein Ct | | |
| | 4) zweistufiges Verfahren | a) mleu7a 2x10^8 Kopien + Mais CDNA 5 ng | 25,54 | | |
| | | | 25,74 | 25,64 | 0,39 |
| | | | 25,65 | | |
| | | b) H₂O in PAP - RT Reaktion | Kein Ct | | |
| | | | Kein Ct | Kein Ct | |
| | | | Kein Ct | | |

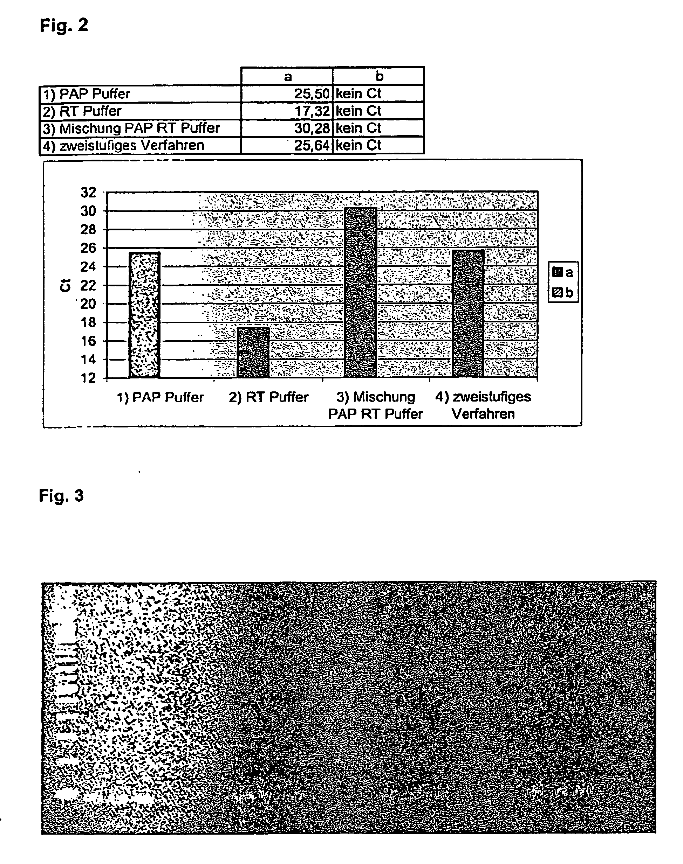

Die Identität der PCR Produkte wurde anschließend mit Hilfe von Agarose-Gelelektrophorese überprüft. Dazu wurden 10 µl jeder PCR-Reaktion auf ein mit Ethidiumbromid gefärbtes 2% Agarosegel geladen und aufgetrennt. Als Größenstandard diente 100bp Lader (Invitrogen, Kata-Iognummer 15628-050). Die Ergebnisse sind in Fig. 3 dargestellt.

### Beispiel 2:

Demonstration der Reproduzierbarkeit und Spezifität eines gekoppelten einstufigen Prozesses von Poly-(A)-Polymerasereaktion und reverser Transkription im gleichen Reaktionsgefäß

In diesem Experiment sollte die Machbarkeit eines gekoppelten einstufigen Prozesses von Poly-(A)-Polymerase Reaktion und reverser Transkription im gleichen Reaktionsgefäß reproduziert werden. Dazu wurde die Effizienz des einstufigen Prozesses von Poly-(A)-Polyrnerasenreaktion und reverser Transkription im gleichen Reaktionsgefäß am Beispiel des Nachweises eines 22-mer RNA Oligonukleotids analysiert. Eine reverse Transkriptionsreaktion für das verwendete Template nach Standard-Bedingungen diente als Kontrolle für die Spezifität des Nachweises.

Zu diesem Zweck wurde der gekoppelte einstufige Prozess unter verschiedenen Bedingungen durchgeführt. Diese waren zu einen der mit Poly-(A)-Polymerase gelieferte Puffer, zum anderen der mit der reversen Transkriptase gelieferte Puffer (Tabelle 7, 8).

**Tabelle 7: Bezeichnung und Komponenten der durchgeführten Reaktionen**

| Bezeichnung | Puffer | Template |
|---|---|---|
| Reaktion 1 | 1 Schritt Verfahren in PAP Puffer | RNA 50 ng + mleu7a 2x10E9 Kopien |
| Reaktion 2 | | RNA 50 ng |
| Reaktion 3 | | negativ Kontrolle :H₂O |
| Reaktion 4 | 1 Schritt Verfahren in RT Puffer | RNA 50 ng + mleu7a 2x10E9 Kopien |
| Reaktion 5 | | RNA 50 ng |
| Reaktion 6 | | negativ Kontrolle :H₂O |
| Reaktion 7 | Standard RT | RNA 50 ng + mleu7a 2x10E9 Kopien |
| Reaktion 8 | | RNA 50 ng |

**Tabelle 8: Zusammensetzung der kombinierten Poly-(A)-Polymerase/Reverse Transkriptions Reaktion und der Standard Reverse Transkriptions Reaktion**

| | Angaben der finalen Konzentrationen | | |
|---|---|---|---|
| Reagenzien | Reaktion 1, 2, 3 PAP Puffer | reaktion 4, 5, 6 RT Puffer | Reaktion 7, 8 Standard RT |
| 5x PAP Puffer | 1 x | | |
| 10x RT Puffer | | 1x | 1x |
| MnCl₂ 25 mM | 2,5 mM | | |
| rATP 10 mM | 1 mM | 1 mM | |
| Poly-(A)-Polymerase 2U/µl | 2U (0,1U/µl) | 2U (0,1U/µl) | |
| dNTP Mix (dA, dT, dG, dC, je 5mM) | 0,5 mM | 0,5 mM | 0,5 mM |
| UniGAPdT Primer 10 µM | 1 µM | 1 µM | 1 µM |
| RNase Inhibitor IO U/µl | 10 U | 10 U | 10 U |
| Sensiscript Reverse Transkriptase | 1 µl | 1 µl | 1 µl |
| RNase freies Wasser | variabel | variabel | variabel |
| mleu7a: Reaktion 1, 4, 7 | 2x10E9 Kopien | 2x 10E9 Kopien | 2x10E9 Kopien |
| RNA : Reaktion 1, 2, 4, 5, 7, 8 | 50 ng | 50 ng | 50 ng |
| Neg. Kontrolle (H₂O anstatt RNA) in Reaktion 3, 6 | H₂O | H₂O | |
| Gesamtvolumen | 20 µl | 20 µl | 20 µl |
| | | | |
| Inkubation | 1 Std. 37°C | | |
| | 5 min 93°C | | |
| | | | |
| Alle Reaktionsansätze wurden anschließend geteilt: Ansätze a) 10 µl wurden entnommen und bei 4°C gelagert; Für alle Ansätze b): Zu 10 µl wurden erneute Uni GAP dT Primer [1 µM ] und 0,5 µl Sensiscript Reverse Transkriptase hinzugefügt und erneut eine Reverse Transkription durchgeführt (1 Std. 37°C), anschließend die Reverse Transkriptase inaktiviert (5 min 93°C). | | | |

Weiterhin wurde eine Standard reverse Transkriptionsreaktion durchgeführt, mit dem Ziel, die Spezifität des Nachweises in der nachfolgenden PCR zu überprüfen (Tabelle 7, 8 siehe oben). Nach der Poly-(A)-Polymerasereaktion und reverse Transkription wurden die Proben geteilt. Jeweils zur Hälfte einer Probe wurde nochmals Uni Gap dT Primer und reverse Transkriptase zugegeben (sieh Tabelle 7, oben). Hierdurch sollte die Möglichkeit ausgeschlossen werden, dass es zu einem geringen Ausmaß Falsch-positive Signale durch ein ungewünschtes Anhängen eines A-Tails an den Uni Gap dT Primer entstehen. Als Template wurde Gesamt-RNA zugegeben, die aus humanem Blut mit einem RNeasy Midi Kit (QIAGEN, Hilden, Deutschland, Kat. No. 75144) isoliert wurde.

Die in den einzelnen Reaktionen eingesetzten Komponenten und ihre Bezeichnungen sind in der Tabelle 7, siehe oben, zusammengefasst. Die nachgewiesenen 22-mer RNA entspricht in ihrer Sequenz der humanen leu7a miRNA (EMBL Acc#: AJ421724) und wird möglicherweise in humanen Blutzellen wie Leukozyten exprimiert. Allerdings werden solche kleinen RNAs bedingt durch die Aufreinigungstechnologie des für die RNA-Isolierung verwendeten RNeasy Verfahrens nur sehr ineffizient aufgereinigt. Das RNeasy Verfahren gewährleistet nur eine effiziente Bindung von RNA's mit einer Größe über 200 Basen an der Silikamembran der RNeasy Säule (QIAGEN RNeasy Midi/Maxi Handbook, 06/2001, S. 9) und damit werden kleine RNA's wie miRNAs stark abgereichert.

Zusätzlich wurde die synthetische 22-mer RNA in einer Konzentration verwendet, die deutlich höher ist als die zu erwartende endogene Kopienzahl. Die Reaktionen wurden wie in Tabelle 8 (sich oben) angegeben zusammengesetzt.

Dazu wurden die in Tabelle 9 angegebenen Reagenzien verwendet.

**Tabelle 9: Materialien für die Poly-A-Reaktion und Reverse Transkription**

| | |
|---|---|
| Poly (A) Polymerase | Ambion; Material Nummer 80U: 2030 |
| Sensiscript RT Kit | Qiagen; Material Nummer 50rxn: 205211 |
| Adenosine 5'-Triphosphate (rATP) | Amersham; Material Nummer 25µmol: 27-2056-01 |
| RNasc Inhibitor | Promega: Material Nummer: N2511 |
| Uni GAP dT Primer | |
| RNA | Leukozyten RNA aus humanem Blut mit einem RNeasy Midi Kit isoliert (QIAGEN, Cat. No.75144) |
| mleu7a RNA Oligonukleotid | 5'-UGA GGU AGU AGG UUG UAU AGU U-3' |

Nach der Inaktivierung der Enzyme (5 min bei 93°C) wurden die Ansätze 1:2 mit Wasser verdünnt und je 2 µl der Ansätze 1a/b bis 8a/b als Template in eine real-time SYBR Green PCR eingesetzt. Der Ansatz der real-time PCR erfolgte in Zweifachansätzen wie in Tabelle 9 (oben) angegeben mit QuantiTect SYBR Green PCR Kit (Katalog Nr. 204143) und den in Tabelle 10 angegebenen Primern.

**Tabelle 10: Komponenten für SYBR Green Real-time PCR**

| | Finale Konzentration |
|---|---|
| 2x SYBR Green PCR Master Mix | 1x |
| Hum Uni Primer 10 µM | 0,5 µM |
| miRNA Primer let7short 10µM | 0,5 µM |
| RNase-free water | variabel |
| PAP-/ RT- Reaktion 1:2 vorverdünnt | 2 µl |
| Finales Reaktionsvolumen | 20 µl |

Die im universellen Tail-Primer Hum Uni Primer enthaltene Sequenz 5'-AAC GAG ACG ACG ACA GAC-3' wurde in US 2003/0186288A1 beschrieben. Das PCR Protokoll bestand aus einer initialen Reaktivierung der im QuantiTect SYBR Green PCR Master Mix enthaltenen HotStarTaq Polymerase für 15 min bei 95°C, gefolgt von 40 Zyklen mit 15 sec 94°C, 30 sec bei 52°C und 30 sec bei 72°C (siehe Tabelle 11).

**Tabelle 11: Materialien für die SYBR Green PCR**

| QuantiTect SYBR Green PCR Kit (200) | Qiagen; Material Nummer: 204143 |
|---|---|
| | |
| Let 7short (spezifischer miRNA Primer) - | 5'-GAG GTA GTA GGT TGT ATA G-3' |
| Hum Uni Primer | 5'-AAC GAG ACG ACG ACA GAC-3' |

Die Aufnahme der Fluoreszenzdaten erfolgte während des 72°C Extensions-Schrittes. Die PCR-Analysen wurden mit einem Applied Biosystems 7500 Fast Real-Time PCR System (Applied Biosystems) in einem Reaktionsvolumen von 20 µl durchgeführt und anschließend eine Schmelzkurvenanalyse durchgeführt.

Die gekoppelte einstufige Poly-(A)-Reaktion und reverse Transkription sind sowohl in Poly-(A)-Polymerase Puffer als auch in RT-Puffer möglich, was anhand der real-time PCR-Analysen deutlich wird. Bevorzugte Pufferbedingungen wurden im Text (siehe oben) bereits angeben. Es zeigen sich große Unterschiede in den erhaltenen Ct-Werten bei Verwendung der unterschiedlichen Puffer.

Die zur Kontrolle der Spezifität durchgeführten Standard Reverse Transkriptionsreaktionen (Reaktionen 3, 6) ohne Poly-(A)-Reaktionen sind alle negativ (kein Ct). Dies lässt den Schluss zu, dass ohne Polyadenylierung der 22-mer RNA (1, 4, 7) bzw. der natürlich vorkommenden miRNA (2, 5, 8) wie erwartet kein Template für eine PCR-Amplifikation vorliegt und daher kein Signal generiert werden kann ("kein Ct").

In den Ansätzen "RT doppelt" wurde nach der ersten Inkubation weiteres RT Enzym und Uni GAP dT Primer zugegeben mit dem Ziel, möglicherweise in der ersten Reaktion Poly-(A)-getailten UniGap dT Primer durch eine RT Reaktion nachweisbar zu machen. Alle diese Ansätze zeigten keinen Ct, d.h. ungewünschte Artefakte sind nicht nachweisbar.

Ungewünschte Artefakte wie Poly-(A)-Tailing des für die cDNA-Synthese verwendeten Primers, sind ebenso nicht nachweisbar.

### Beispiel 3:

### Nachweis verschiedener miRNAs mit Hilfe des erfindungsgemäßen Verfahrens.

In diesem Experiment sollte demonstriert werden, dass aus einer Template cDNA , die mit dem erfindungsgemäßen Verfahren mit Poly-(A)-Reaktion gemeinsamer reverser Transkription synthetisiert wurde, mehrere Targets durch miRNA spezifische PCR Primer nachgewiesen werden können. Hierfür wurde Verfahren mit 293 RNA als Template durchgeführt. Eine Reverse Transkriptions Reaktion für das verwendete Template nach Standard-Bedingungen diente als Kontrolle für die Spezifität des Nachweises.

In der anschließenden SYBR Green PCR wurde insgesamt jeweils einer von 4 verschiedenen spezifischen Primer für miRNA's zusammen mit dem Tail spezifischen Primer eingesetzt. Zusätzlich wurde ein am 3' Ende des humanen β-Actin Transkripts lokalisierter Primer zusammen mit einem Tail spezifischen Primer verwendet, um die Effizienz der Poly-A-Reaktion und Reversen Transkription zu prüfen.

Für die Poly-A-Reaktion und Reversen Transkription (PAP + RT Reaktion) wurden die Reagenzien aus Tabelle 13 wie in Tabelle 16 angegeben zusammenpipettiert.

**Tabelle 13: Materialien für die Poly A Reaktion und Reverse Transkription**

| | |
|---|---|
| Poly (A) Polymerase | Ambion; Material Nummer 80U: 2030 |
| Sensiscript RT Kit | Qiagen; Material Nummer 50rxn: 205211 |
| Adenosine 5'-Triphosphate (rATP) | Amersham; Material Nummer 25µmol: 27-2056-01 |
| RNase Inhibitor | Promeea: Material Nummer: N2511 |
| Uni GAP dT Primer | |
| 293 RNA: aus humaner Zell-Linie 293 (ATCC Nummer: CRL-1573) | Mit Qiagen RNeasy Midi Kit isoliert |

**Tabelle 16: PAP + RT Reaktion**

| Reagenzien | Finale Konzentration |
|---|---|
| 10x Buffer RT | 1 x |
| rATP 10 mM | 1 mM |
| Poly A Polymerase 2U/ µl | 2 U |
| dNTP Mix (ATGC je 5mM ) | 0,5 mM |
| Uni GAP dT Primer 10 µM | 1 µM |
| RNase Inhibitor 10 U/ µl | 10 U |
| Sensiscript Reverse Transkriptase | 1 µl |
| RNase freies Wasser | variabel |
| a) 293 RNA 20 ng/ µl | 5 µl (100 ng) |
| b) H₂O für neg Kontrolle | 5 µl |
| Gesamtvolumen | 20 µl |
| Inkubation | 1 Std. 37°C |
| | 5 min 93°C |

In Reaktion a) 293 RNA und in Reaktion b) Wasser als Negativkontrolle (neg Kontrolle) zugegeben. Als Kontrolle für die Spezifität des Nachweises wurde eine Standard Reverse Transkriptions Reaktion mit den in Tabelle 14 angegebenen Reagenzien anhand des Schemas in Tabelle 17 angesetzt.

**Tabelle 14: Materialien für die Reverse Transkription**

| | |
|---|---|
| Sensiscript RT Kit RNase Inhibitor | Qiagen; Material Nummer 50rxn: 205211 Promega; Material Nummer: N2511 |
| Uni GAP dT Primer | |
| 293 RNA | Mit Qiagen RNeasy Midi Kit isoliert |

**Tabelle 17: Standard RT Reaktion**

| Reagenzien | 2.) RT Reaktion |
|---|---|
| 10x Buffer RT | 1x |
| dNTPMix(ATGC je 5mM ) | 0,5 mM |
| Uni GAP dT Primer 10 µM | 1 µM |
| RNase Inhibitor 10 U/ µl | 10 U |
| Sensiscript Reverse Transkriptase | 1 µl |
| RNase freies Wasser | variabel |
| c) 293 RNA 20 ng/ µl | 5 µl (100 ng) |
| d) H₂O für neg Kontrolle | 5 µl |
| Gesamtvolumen | 20 µl |
| Inkubation | 1 Std. 37°C |
| | 5 min 93°C |

In Reaktion in Reaktion c) 293 RNA und in Reaktion d) Wasser als Negativkontrolle (neg Kontrolle) zugegeben. Die Proben wurden anschließend für eine Stunde bei 37°C inkubiert. Um die Reaktion zu stoppen, wurden die Reaktionen für 5 min bei 93°C inkubiert, durch diesen Temperaturschritt werden die Enzyme inaktiviert.

Nach der Inaktivierung der Enzyme wurden die Ansätze 1:2 mit Wasser verdünnt und je 2 µl der Ansätze a) bis d) als Template in eine real-time SYBR Green PCR eingesetzt. Die Materialien für die PCR sind in Tabelle 15 angegeben.

**Tabelle 15: Materialien für die SYBR Green PCR**

| QuantiTect SYBR Green PCR Kit (200) | Qiagen; Material Nummer: 204143 |
|---|---|
| let 7short (spezifischer miRNA Primer) | 5'-GAG GTA GTA GGT TGT ATA G-3' |
| hsa-miR-24 (spezifischer miRNA Primer) | 5'-TGG CTC AGT TCA GCA GGA-3' |
| hsa-miR-15a (spezifischer miRNA Primer) | 5'-TAG CAG CAC ATA ATG GTT T-3' |
| hsa-miR-16 (spezifischer miRNA Primer) | 5'-TAG CAG CAC GTA AAT ATT G-3' |
| ß-Actin 3' Primer | |
| Hum Uni Primer | 5'-AAC GAG ACG ACG ACA GAC-3' |

Es wurden 10 verschieden Reaktionsansätze pipettiert. In Reaktion 1-5 (Tabelle 18) wurden jeweils der miRNA spezifische bzw. ß-Actin 3' Primer und der Tail-Primer (Hum Uni) eingesetzt.

**Tabelle 18: SYBR GREEN PCR Reaktion 1-5**

| Komponenten für SYBR Green PCR | Finale Konzentration |
|---|---|
| 2x SYBR Green PCR Master Mix | 1x |
| Hum Uni Primer 10 µM | 0,5 µM |
| je ein spezifischer miRNA Primer (10 µM) | 0,5 µM |
| let 7short | |
| hsa-miR-24 | |
| hsa-miR-15a | |
| hsa-miR-16 | |
| bzw. ß-Actin 3' Primer | |
| RNase-free water | variabel |
| PAP + RT Reaktion a) b) 1:2 vorverdünnt | 2 µl (5 ng) |
| Standard RT Reaktion c) d ) 1:2 vorverdünnt | 2 µl (5 ng) |
| bzw. H₂O als neg Kontrolle | 2 µl |
| Finales Reaktionsvolumen | 20 µl |

In Reaktion 6-10 (Tabelle 19) wurde jeweils nur ein Primer eingesetzt, entweder der miRNA spezifische Primer oder der Tail-spezifische Primer.

In Reaktion 6-10 (Tabelle 19) wurde jeweils nur ein Primer eingesetzt, entweder der miRNA spezifische Primer oder der Tail-spezifische Primer.

**Tabelle 19: Kontrolle mit nur einem Primer Reaktion 6-10**

| Komponenten für SYBR Green PCR | Finale Konzentration |
|---|---|
| 2x SYBR Green PCR Master Mix | 1x |
| je ein spezifischer miRNA Primer (10 µM) | 0,5 µM |
| let 7short | |
| hsa-miR-24 | |
| hsa-miR-15a | |
| hsa-miR-16 | |
| jeweils mit 3'Hum Uni Primer | |
| RNase-free water | variabel |
| PAP + RT Reaktion a) b) 1:2 vorverdünnt | 2 µl (5 ng) |
| Standard RT Reaktion c) d) 1:2 vorverdünnt | 2 µl (5 ng) |
| bzw. H₂O als neg Kontrolle | 2 µl |
| Finales Reaktionsvolumen | 20 µl |

Die Reihenfolge, in der die Primer in die Reaktionen eingesetzt wurden geht aus Tabelle 20 hervor. Der Ansatz der real-time PCR erfolgte in Zweifachansätzen.

**Tabelle 20: Primer**

| | Primer |
|---|---|
| Reaktion 1 | ß-Actin 3' Primer + Hum Uni |
| Reaktion 2 | let 7 short + Hum Uni |
| Reaktion 3 | hsa-miR-24 + Hum Uni |
| Reaktion 4 | hsa-miR-15^{a} + Hum Uni |
| Reaktion 5 | hsa-miR-16 + Hum Uni |
| Reaktion 6 | Hum Uni |
| Reaktion 7 | let 7short |
| Reaktion 8 | hsa-miR-24 |
| Reaktion 9 | hsa-miR-15a |
| Reaktion 10 | hsa-miR-16 |

Die im universellen Tail-Primer Hum Uni Primer enthaltene Sequenz AAC GAG ACG ACG ACA GAC wurde in US2003/0186288A1 beschrieben.

Das PCR Protokoll bestand aus einer initialen Reaktivierung der im QuantiTect SYBR Green PCR Master Mix enthaltenen HotStarTaq Polymerase für 15 min bei 95°C, gefolgt von 40 Zyklen mit 15 sec 94°C, 30 sec bei 52°C und 30 sec bei 72°C (siehe Tabelle 21). Die Aufnahme der Fluoreszenzdaten erfolgte während des 72°C Extensions-Schrittes. Die PCR-Analysen wurden mit einem Applied Biosystems 7000 Fast Real-Time PCR System (Applied Biosystems) in einem Reaktionsvolumen von 20 µl durchgeführt und anschließend eine Schmelzkurvenanalyse durchgeführt.

**Tabelle 21: 3 Schritt PCR Protokoll**

| | | |
|---|---|---|
| PCR Initial Reaktivierung | 15 min 95°C | |
| Denaturierung | 15 sec 94°C | 40x |
| Annealing | 30 sec 52°C | |
| Extension | 30 sec 72°C | |
| Schmelzkurve | | |

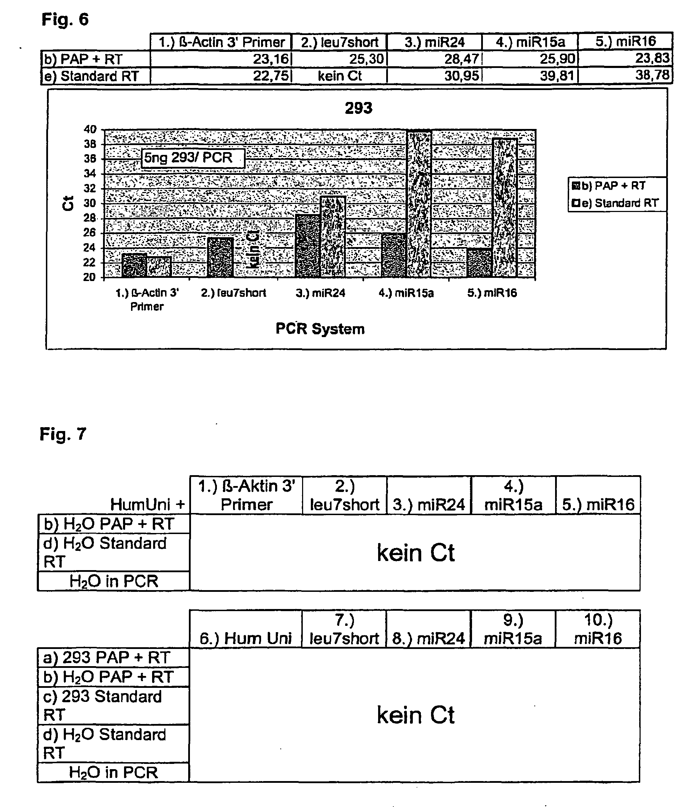

Es zeigt sich, dass die Effizienz der unter Standard-Bedingungen durchgeführten Reversen Transkription und des erfindungsgemäßen Verfahrens für das exemplarisch ausgewählte ß-Actin System vergleichbar ist, was an vergleichbaren Ct Werten in der real-time PCR deutlich wird (Fig. 6).

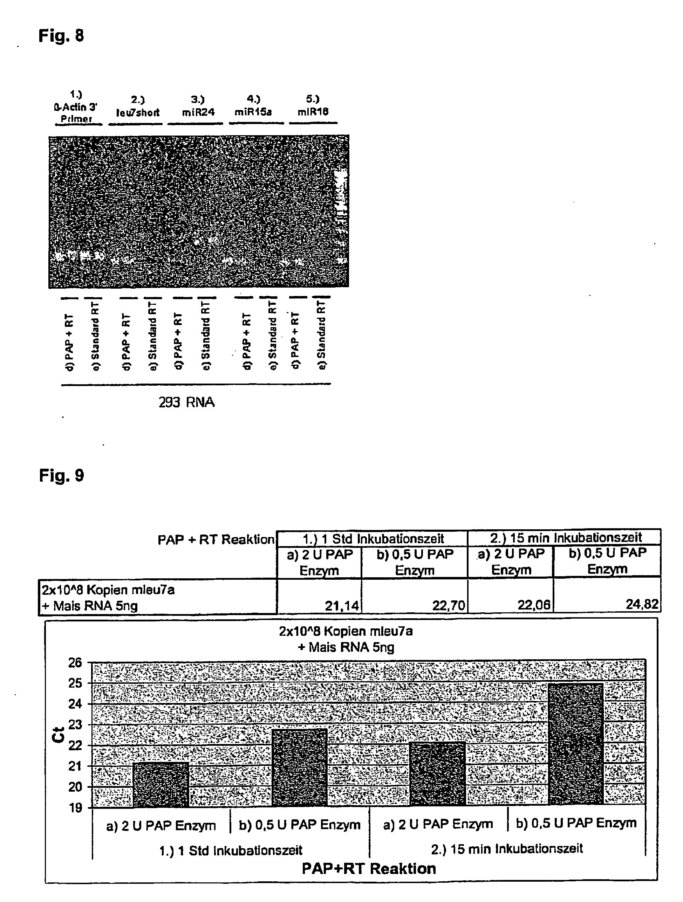

Die real-time PCR liefert bei Verwendung der unter Standard-Bedingungen hergestellten cDNA sehr hohe Ct Werte von über 38, die bei einer mit SybrGreen durchgeführten real-time PCR eine sehr gute Spezifität des Nachweises bedeuten (Fig. 6). Bei der Agarosegel-Analyse der PCR Produkte wurden PCR Produkte der erwarteten Größe detektiert (Fig. 8), bzw. es wurde kein Produkt bei Verwendung der unter Standard-Bedingungen hergestellten cDNA detektiert.

Eine Ausnahme stellt das miR24 Produkt dar. Hier tritt bei Verwendung der unter Standard-Bedingungen hergestellten cDNA ein PCR Produkt der falschen Größe auf (Fig. 8, siehe auch Fig. 6). Dieses Produkt ist nicht nachweisbar, sobald eine cDNA eingesetzt wird, die mit Hilfe des erfindungsgemäßen Verfahrens hergestellt wurde (Fig. 8).

Alle Kontrollreaktionen bei denen Wasser anstatt Template RNA in der Reversen Transkription bzw. des erfindungsgemäßen Verfahrens eingesetzt wurde zeigen kein Signal, d.h. es wurde kein Ct Wert in den betreffenden real-time PCRs erhalten (Fig. 7, oberer Teil). Das gleiche gilt auch für Reaktionen, bei denen lediglich ein Primer eingesetzt wurde (Fig. 7, oberer Teil). Ebenfalls kein Ct Wert wurde für Negativkontrollen, bei denen Wasser anstatt cDNA in der PCR eingesetzt wurde, erhalten (Fig. 7).

### Beispiel 4:

Nachweises von miRNA mit Hilfe des gekoppelten, einstufigen Prozesses von Poly-A-Reaktion und Reverser Transkription und anschließendem Nachweis der generierten cDNA über real-time PCR mit Tail-spezifischer Sonde.

In diesem Experiment wurde eine real-time PCR durchgeführt, bei der eine Taqman Sonde eingesetzt wurde, die eine spezifische Bindungsstelle am Tail-Primer (Uni Gap dT) hat. Der Nachweis über eine Sonde stellt eine denkbare alternative für den Nachweis über SYBR Green real-time PCR dar. Die Verwendung der Sonde bietet die zusätzliche Möglichkeit einer Multiplex PCR, d.h. einer Co-Amplifikation einer oder mehrerer zusätzlicher Ziel-Nukleinsäuren, wie einer internen Kontrolle, die z.B. ein House-Keeping Gen sein kann.

Für die Poly-A-Reaktion und Reversen Transkription (PAP + RT Reaktion) wurden die Reagenzien aus Tabelle 22 wie in Tabelle 24 angegeben zusammenpipettiert.

**Tabelle 22: Materialien für die Poly A Reaktion und Reverse Transkription**

| | |
|---|---|
| | |
| Poly (A) Polymerase | Ambion; Material Nummer 80U: 2030 |
| Sensiscript RT Kit | Qiagen; Material Nummer 50rxn: 205211 |
| Adenosine 5'-triphosphate (rATP) | Amersham; Material Nummer 25µmol: 27-2056-01 |
| RNase Inhibitor | Promega: Material Nummer: N2511 |
| Uni GAP dT Primer | |
| mleu7a Oligonukleotid | 5'-UGA GGU AGU AGG UUG UAU AGU U-3' |

**Tabelle 24: PAP + RT Reaktion**

| Reagenzien | Finale Konzentration |
|---|---|
| 10x Buffer RT | 1 x |
| rATP 10 mM | 1 mM |
| Poly A Polymerase 2U/ µl | 2 U |
| dNTP Mix (ATGC je 5mM ) | 0,5 mM |
| Uni GAP dT Primer 10 µM | 1 µM |
| RNase Inhibitor 10 U/ µl | 10 U |
| Sensiscript Reverse Transkriptase | 1 µl |
| RNase freies Wasser | variabel |
| mleu7 (10^9 Kopien/µl) | 2 µl (2x10^9 Kopien) |
| Gesamtvolumen | 20 µl |
| Inkubation | 1 Std 37°C |
| | 5 min 93 °C |

Anschließend wurde der Reaktionsansatz bei 37°C inkubiert, gefolgt von einer Inaktivierung der Enzyme für 5 min auf 93°C.

Nach der Inaktivierung der Enzyme wurden 2 µl des unverdünnten Ansatzes als Template in eine real-time PCR eingesetzt, die zur Detektion eine Taqman Sonde enthielt. Die Materialien für die PCR sind in Tabelle 23 angegeben, und wurden wie in Tabelle 25 angegeben zusammcnpipettiert.

**Tabelle 23: Materialien für die QT Probe PCR**

| QuantiTect Probe PCR Kit (200) | Qiagen Material Nr.: 204343 |
|---|---|
| let 7short (spezifischer miRNA Primer) | 5'-GAG GTA GTA GGT TGT ATA G-3' |
| Hum Uni Primer | 5'-AAC GAG ACG ACG ACA GAC-3' |
| Hum Uni Sonde | |
| | 5' Reporter Farbstoff HEX |
| | 3' Quencher: Black Hole Quencher 1 |

**Tabelle 25: QuantiTect Probe PCR**

| Komponenten für QuantiTect Probe PCR | Finale Konzentration |
|---|---|
| 2x QuantiTect Probe PCR Master Mix | 1 x |
| Hum Uni Primer 10 µM | 0,5 µM |
| let 7short (spezifischer miRNA Primer) | 0,5 µM |
| RNase-free water | Variabel |
| PAP + RT Reaktion unverdünnt | 2 µl (2x10^8 Kopien) |
| Finales Reaktionsvolumen | 20 *µl |

Der Ansatz der real-time PCR erfolgte in Zweifachansätzen.
Die im universellen Tail-Primer Hum Uni Primer enthaltene Sequenz AAC GAG ACG ACG ACA GAC wurde in US2003/0186288A1 beschrieben. Die Sequenz der Taqman Sonde wurde dem humanen GAPDH Gen Lokus entnommen, sie ist nicht in US2003/0186288A1 enthalten.

Das PCR Protokoll bestand aus einer initialen Reaktivierung der im QuantiTect Probe PCR Master Mix enthaltenen HotStarTaq Polymerase für 15 min bei 95°C, gefolgt von 45 Zyklen mit 15 sec 94°C und 30 sec bei 52°C (siehe Tabelle 26).

**Tabelle 26: 2 Schritt PCR Protokoll**

| | | |
|---|---|---|
| PCR Initiale Reaktivierung | 15 min 95°C | |
| Denaturierung | 15 sec 94°C | 45x |
| Annealing | 30 sec 52°C | |

Die Aufnahme der Fluoreszenzdaten erfolgte während des 52°C Annealing-Schrittes. Die PCR-Analysen wurden mit einem 7700 Sequence Detection System (Applied Biosystems) in einem Reaktionsvolumen von 20 µl durchgeführt. Die PCR-Ergebnisse sind in Tabelle 27 gezeigt.

**Tabelle 27: PCR Ergebnisse**

| mleu7a | Ct | Ct Mittel | CV in % |
|---|---|---|---|
| unverdünnt 2x10^8 | 20,24 | 20,31 | 0,45 |
| | 20,37 | | |

Ein Nachweis mit Hilfe einer für den Tail spezifischen Sonde ist möglich und liefert das zu erwartende Ergebnis.

### Beispiel 5:

Effekt von Poly-A-Polymerase Konzentration und Inkubationszeit auf den gekoppelten, einstufigen Prozesses von Poly-A-Reaktion und Reverser Transkription.

In diesem Versuch wurden zwei Konzentrationen Poly-A-Polymerase (2 U bzw. 0,5 U) für jeweils 15 min oder 1 h im erfindungsgemäßen Verfahren eingesetzt. Alle Bedingungen wurden jeweils in RT Puffer (Qiagen) und Poly-A-Polymerase Puffer getestet.

Für die Poly-A-Reaktion und Reverse Transkription (PAP + RT Reaktion) wurden die Reagenzien aus Tabelle 28 wie in Tabelle 30 angegeben zusammenpipettiert (Reaktion a) 2 U Poly-A-Polymerase, Reaktion b) 0,5 U Poly-A-Polymerase).

**Tabelle 28: Materialien für die Poly A Reaktion und Reverse Transkription**

| | |
|---|---|
| Poly (A) Polymerase | Ambion; Material Nummer 80U: 2030 |
| Sensiscript RT Kit | Qiagen; Material Nummer 50rxn: 205211 |
| Adenosine 5'-Triphosphate (rATP) | Amersham; Material Nummer 25µmol: 27-2056-01 |
| RNase Inhibitor | Promesa: Material Nummer: N2511 |
| Uni GAP dT Primer | |
| Mais RNA | Aus 1g gemörserten Maisblättern mit Qiagen RNeasy Mini Kit (Cat. No. 74106) nach Plant Protokoll. |
| mleu7a Oligonukleotid | 5'-UGA GGU AGU AGG UUG UAU AGU U-3' |

**Tabelle 30: PAP + RT Reaktion**

| Reagenzien | Finale Konzentration |
|---|---|
| 10x Buffer RT | 1 x |
| rATP 10 mM | 1mM |
| Poly A Polymerase 2U/ µl | a) 2 U |
| | b) 0,5 U |
| dNTP Mix ( ATGC je 5mM ) | 0,5 mM |
| Uni GAP dT Primer 10 µM | 1 µM |
| RNase Inhibitor 10 U/ µl | 10U |
| Sensiscript Reverse Transkriptase | 1 µl |
| RNase freies Wasser | variabel |
| mleu7a 10^9 Kopien/µl | 2 µl (2x10^9 Kopien) |
| Mais RNA 25 ng/µl | 2 µl ( 50 ng) |
| Gesamtvolumen | 20 µl |
| 1.) Inkubation | 1 Std 37°C |
| | 5 min 93°C |
| 2.) Inkubation | 15 min 37°C |
| | 5 min 93°C |

Anschließend wurden die Proben bei 37°C inkubiert (1. 1 Std./ 2. 15 min). Anschließend wurden die Reaktionen für 5 min auf 93°C erhitzt und dadurch die Enzyme inaktiviert.

Anschließend wurden je Reaktion jeweils 2 µl unverdünnt in eine SYBR Green PCR eingesetzt. Die Reaktionen wurden in Doppelbestimmungen getestet. Hierfür wurden die Reagenzien aus Tabelle 29 wie in Tabelle 31 angegeben zusammenpipettiert und anschließend die PCR wie in Tabelle 32 angegeben durchgeführt.

**Tabelle 29: Materialien für die SYBR Green PCR**

| QuantiTect SYBR Green PCR Kit (200) | Qiagen; Material Nummer: 204143 |
|---|---|
| Let 7short (spezifischer miRNA Primer) | 5'-GAG GTA GTA GGT TGT ATA G-3' |
| Hum Uni Primer | 5'-AAC GAG ACG ACG ACA GAC-3' |

**Tabelle 31: SYBR GREEN PCR**

| Komponenten für SYBR Green PCR | Finale Konzentration |
|---|---|
| 2x SYBR Green PCR Master Mix | 1x |
| Hum Uni Primer 10 µM | 0,5 µM |
| let 7short (spezifischer miRNA Primer) | 0,5 µM |
| RNase-free water | variabel |
| PAP + RT Reaktion 1a) b)/ 2a) b) | 2 µl (2x10^8 Kopien) |
| Finales Reaktionsvolumen | 20 µl |

**Tabelle 32: 3 Schritt PCR Protokoll**

| | | |
|---|---|---|
| PCR Initiale Reaktivierung | 15 min 95°C | |
| Denaturierung | 15 sec 94°C | 45x |
| Annealing | 30 sec 52°C | |
| Extension | 30 sec 70°C | |
| Schmelzkurve | | |

Das PCR Protokoll bestand aus einer initialen Reaktivierung der im QuantiTect SYBR Green PCR Master Mix enthaltenen HotStarTaq Polymerase für 15 min bei 95°C, gefolgt von 40 Zyklen mit 15 sec 94°C, 30 sec bei 52°C und 30 sec bei 72°C (siehe Tabelle 32 oben). Die Aufnahme der Fluoreszenzdaten erfolgte während des 72°C Extensions-Schrittes. Die PCR-Analysen wurden mit einem Applied Biosystems 7000 Real-Time PCR System (Applied Biosystems) in einem Reaktionsvolumen von 20 µl durchgeführt und anschließend eine Schmelzkurvenanalyse durchgeführt.

Es zeigt sich eine Abhängigkeit der Effizienz des einstufigen Prozesses von Poly-A-Reaktion und Reverser Transkription sowohl von der Konzentration der Poly-A-Polymerase als auch von der Inkubationszeit (seihe Fig. 9).

### Beispiel 6:

Durchführung des erfindungsgemäßen Verfahrens mit verschiedenen Reversen Transkriptasen.

Das erfindungsgemäße Verfahren wurde mit insgesamt fünf verschiedenen Reversen Transkriptasen (siehe Tabelle 35) im Buffer RT (Qiagen) (Reaktion 1-5), bzw. zusätzlich zum Vergleich jeweils im mit der Reversen Transkriptase gelieferten Puffer angesetzt (Reaktion 6-9).

**Tabelle 35: Verwendete Reverse Transkriptasen und Puffer**

| | |
|---|---|
| 1.) AMV Reverse Transkriptase | AMV Reverse Transcriptase 5 x Reaction Buffer |
| 2.) SuperScript III Reverse Transkriptase | |
| 3.) HIV Reverse Transkriptase | 5 x First- Strand Buffer |
| 4.) M-MuLV Reverse Transkriptase | 10 x First Strand Synthesis Buffer |
| 5.) Sensiscript Reverse Transkriptase | 10 x Reverse Transcriptase Reaction Buffer |
| | 10x Buffer RT |

Für den einstufigen Prozess von Poly-A-Reaktion und Reverser Transkription (PAP + RT Reaktion) wurden die Reagenzien aus Tabelle 33 für Reaktion 1-5 (Reaktionspuffer: Buffer RT (Qiagen) wie in Tabelle 36, und für Reaktion 6-9 (Weitere Reverse Transkriptasen, jeweils im mitgelieferten Puffer) wie in Tabelle 37 angegeben zusammenpipettiert.

**Tabelle 33: Materialien für die Poly A Reaktion und Reverse Transkription**

| | |
|---|---|
| Poly (A) Polymerase | Ambion; Material Nummer 80U: 2030 |
| AMV Reverse Transcriptase | Promega; Material Nummer 300 U: M5101 |
| SuperScript III Reverse Transcriptase | Invitrogen; Material Nummer 10.000 U: 18080-044 |
| HIV Reverse Transcriptase | Ambion; Material Nummer 500 U: #2045 |
| M-MuLV Reverse Transcriptase | BioLabs; Material Nummer 10.000 U: M0253S |
| Sensiscript RT Kit | Qiagen; Material Nummer 50 rxns: 205211 |
| Adenosine 5'-Triphosphate (rATP) | Amersham; Material Nummer 25µmol : 27-2056-01 |
| RNase Inhibitor | Promega; Material Nummer: N2511 |
| Uni GAP dT Primer | |
| Mais RNA | Aus 1g gemörserten Maisblättern mit Qiagen RNeasy Mini Kit (Cat. No. 74106) Plant Protokoll, 10 facher upscale (Maxi shredder und Säule) siehe oben |
| mleu7a Oligonukleotid | 5'-UGA GGU AGU AGG UUG UAU AGU U-3' |

**Tabelle 36: PAP + RT Reaktion in Buffer RT (Qiagen)**

| Reagenzien | Finale Konzentration |
|---|---|
| 10x Buffer RT | 1 x |
| rATP 10 mM | 1 nM |
| Poly A Polymerase 2U/ µl | 1 U |
| dNTP Mix ( ATGC je 5mM ) | 0,5 mM |
| Uni GAP dT Primer 10 µM | 1 µM |
| RNase Inhibitor 10 U/ µl | 10 U |
| 1.) AMV Reverse Transkriptase | 24 U |
| 2.) SuperScript III Reverse Transkriptase | 10 U |
| 3.) HIV Reverse Transkriptase | 1 U |
| 4.) M-MuLV Reverse Transkriptase | 10 U |
| 5.) Sensiscript Reverse Transkriptase | 1 µl |
| RNase freies Wasser | variabel |
| mleu7a 10^9 Kopien/µl | 2 µl (2x10^9 Kopien) |
| Mais RNA 20 ng/µl | 2 µl (40 ng) |
| Gesamtvolumen | 20 µl |
| Inkubation | 1 Std 37°C |
| | 5 min 93°C |

**Tabelle 37: PAP + RT Reaktion im mit der Reversen Transkriptase gelieferten Puffer**

| Reagenzien | Finale Konzentration |
|---|---|
| 6.) AMV Reverse Transcriptase 5 x Reaction Buffer | 1 x |
| | 1 x |
| 7.) 5 x First- Strand Buffer | 1 x |
| 8.) 10 x First Strand Synthesis Buffer | 1 x |
| 9.) 10 x Reverse Transcriptase Reaction Buffer | |
| rATP 10 mM | 1 mM |
| Poly A Polymerase 2U/ µl | 1 U |
| dNTP Mix (ATGC je 5mM) | 0,5 mM |
| Uni GAP dT Primer 10 µM | 1 µM |
| RNase Inhibitor 10 U/ µl | 10 U |
| 6.) AMV Reverse Transkriptase | 24 U |
| 7.) SuperScript III Reverse Transkriptase | 10 U |
| 8.) HIV Reverse Transkriptase | 1 U |
| 9.) M-MuLV Reverse Transkriptase | 10 U |
| RNase freies Wasser | variabel |
| mleu7a 10^9 Kopien/µl | 2 µl (2x10^9 Kopien) |
| Mais RNA 20 ng/µl | 2 µl (40 ng) |
| Gesamtvolumen | 20 µl |
| Inkubation | 1 Std 37°C |
| | 5 min 93°C |

Anschließend wurden die Proben 1 h bei 37°C inkubiert. Danach wurden die Reaktionen für 5 min auf 93°C erhitzt und dadurch die Enzyme inaktiviert.

In der Folge wurden je Reaktion jeweils 2 µl in eine SYBR Green PCR eingesetzt. Die Reaktionen wurden in Doppelbestimmungen getestet. Hierfür wurden die Reagenzien aus Tabelle 34 wie in Tabelle 38 angegeben zusammenpipettiert, und die PCR wie in Tabelle 39 angegeben durchgeführt.

**Tabelle 34: Materialien für die SYBR Green PCR**

| QuantiTect SYBR Green PCR Kit (200) | Qiagen; Material Nummer: 204143 |
|---|---|
| Let 7short (spezifischer miRNA Primer) | 5'-GAG GTA GTA GGT TGT ATA G-3' |
| Hum Uni Primer | 5'-AAC GAG ACG ACG ACA GAC-3' |

**Tabelle 38: SYBR GREEN PCR**

| Komponenten für SYBR Green PCR | Finale Konzentration |
|---|---|
| 2x SYBR Green PCR Master Mix | 1x |
| Hum Uni Primer 10 µM | 0,5 µM |
| let 7short (spezifischer miRNA Primer) | 0,5 µM |
| RNase-free water | variabel |
| PAP + RT Reaktion unverdünnt | 2 µl (2x10^8 Kopien) |
| Finales Reaktionsvolumen | 20 µl |

**Tabelle 39: 3 Schritt PCR Protokoll**

| | | |
|---|---|---|
| PCR Initiale Reaktivierung | 15 min 95°C | |
| Denaturierung | 15 sec 94°C | 45x |
| Annealing | 30 sec 52°C | |
| Extension | 30 sec 70°C | |
| Schmelzkurve | | |

Das PCR Protokoll bestand aus einer initialen Reaktivierung der im QuantiTect SYBR Green PCR Master Mix enthaltenen HotStarTaq Polymerase für 15 min bei 95°C, gefolgt von 40 Zyklen mit 15 sec 94°C, 30 sec bei 52°C und 30 sec bei 72°C (siehe Tabelle 39). Die Aufnahme der Fluoreszenzdaten erfolgte während des 72°C Extensions-Schrittes. Die PCR-Analysen wurden mit einem Applied Biosystems 7000 Real-Time PCR System (Applied Biosystems) in einem Reaktionsvolumen von 20 µl durchgeführt und anschließend eine Schmelzkurvenanalyse durchgeführt.

Die verwendeten Mengen der Reversen Transkriptasen wurden für Standard Reverse Transkriptions Reaktionen optimiert, was eine wahrscheinliche Erklärung für die beobachteten Unterschiede im Ct Wert sein kann.

### Beispiel 7:

Demonstration der Machbarkeit eines gekoppelten dreistufigen Prozesses von Poly-A-Reaktion, reverser Transkription und PCR im gleichen Reaktionsgefäß; Effekt verschiedener Zusätze auf die Effizienz des Nachweises eines 22-mer RNA-Oligonukleotids

In diesem Experiment sollte die Machbarkeit eines gekoppelten dreistufigen Prozesses von Poly-(A)-Polymerase Reaktion, reverser Transkription und PCR im gleichen Reaktionsgefäß demonstriert werden. Zu diesem Zweck wurde der gekoppelte dreistufige Prozess unter den folgenden Bedingungen mit den angegebenen Zusätzen durchgeführt.

Als Kontrolle wurde die Reaktion in einem zweistufigen Verfahren in Anlehnung an die Fig. 1 B durchgeführt.

Für den dreistufigen Prozess von Poly-A-Reaktion, Reverser Transkription und PCR (PAP + RT Reaktion + PCR) wurden die Materialien aus Tabelle 40 wie in Tabelle 41 angegeben zusammengesetzt.

**Tabelle 40: Materialien für die Poly A Reaktion, Reverse Transkription und PCR**

| | |
|---|---|
| Poly (A) Polymerase | Epicentre Biotechnologies; Material Nummer 400U: PAP5104 |
| QuantiTect Multiplex RT-PCR Kit | Qiagen; Material Nummer 200 rxns: 204643 |
| Adenosine 5'-Triphosphate (rATP) | Amersham; Material Nummer 25µmol: 27-2056-01 |
| dNTP Mix (ATGC je 10mM) | Amersham; Material Nummer'Qiagen Intern 1007430 |
| RNase Inhibitor | Promega; Material Nummer: N2511 |
| Uni GAP dT Primer | |
| Mais RNA | Aus 1g gemörserten Maisblättern mit Qiagen RNeasy Mini Kit (Cat. No. 74106) Plant Protokoll, 10 facher upscale (Maxi shredder und Säule) siehe oben |
| mleu7a Oligonukleotid | 5'-UGA GGU AGU AGG UUG UAU AGU U-3' |
| Let 7short (spezifischer miRNA Primer) | 5'-GAG GTA GTA GGT TGT ATA G-3' |
| Hum Uni Primer | 5'-AAC GAG ACG ACG ACA GAC-3' |
| GAPDH-TM-HEX_BHQ | 5'HEX-CAA GCT TCC CGT TCT CAG CC-BHQ 3' |
| Poly A RNA Random N8 Primer Oligo dT 12 | Amersham Biosciences Material Nummer 27-4110-01, gelöst mit 25µg/µl in RNase freiem Wasser NNNNNNNN TTTTTTTTTTTT-3' Phosphat |

**Tabelle 41: PAP + RT Reaktion + PCR mit Zusätzen in QuantiTect Multiplex RT-PCR Master Mix (Qiagen)**

| Reagenzien | Finale Konzentration |
|---|---|
| 2x QuantiTect Multiplex RT-PCR Master Mix | 1 x |
| rATP 10 mM | 100 µM |
| Poly A Polymerase 4U/ µl | 1 U |
| dNTP Mix (ATGC je 10mM) | 0,5 mM |
| Uni GAP dT Primer 10 µM | 0,05 µM |
| RNase Inhibitor 40 U/ µl | 10 U |
| QuantiTect Multiplex RT Mix | 0.2 µl |
| Hum Uni Primer 10 µM | 0,5 µM |
| let 7short (spezifischer miRNA Primer) | 0,5 µM |
| GAPDH-TM-HEX BHQ | 0,2µM |
| RNase freies Wasser | variabel |
| Poly A RNA 25µg/µl | 10ng/µl |
| N8 Random Primer | 0,05µM |
| Oligo dT12 | 5µM |
| mleu7a 10^9 Kopien/µl | 2 µl (2x10⁹ Kopien) |
| Mais RNA 20 ng/µl | 2 µl (40 ng) |
| Gesamtvolumen | 20 µl |

Die Reaktionen wurden in Dreifachbestimmungen getestet. Hierfür wurden die Reagenzien aus Tabelle 40 wie in Tabelle 41 angegeben zusammenpipettiert, und die Reaktion wie in Tabelle 42 angegeben durchgeführt.

**Tabelle 42: Reaktionsprotokoll der "3in1" Reaktion**

| | | |
|---|---|---|
| Poly A Reaktion und Reverse Transkription | 45 min 37°C | |
| | 15min 50°C | |
| PCR Initiale Reaktivierung | 15 min 95°C | |
| Denaturierung | 15 sec 94°C | 45x |
| Annealing/ Extension | 30 sec 52°C | |

Das Reaktionsprotokoll bestand zuerst aus Bedingungen für die kombinierte Reaktion von Poly-A Polymerase und reverser Transkription mit dem QuantiTect Multiplex Reverse Transcriptase Mix (45 min 37°C und 15 min 50°C), Darauf folgte eine Inkubation für 15 min bei 95°C, mit dem Ziel die Poly-A-Polymerase und Reverse Transkriptase zu inaktivieren, sowie die im QuantiTect Multiplex RT-PCR Master Mix enthaltene HotStarTaq DNA Polymerase zu aktivieren. Es folgten 45 PCR Zyklen mit 15 sec bei 94°C und 30 sec bei 52°C (siehe Tabelle 43) um die generierte let7a-cDNA in einer real-time PCR zu amplifizieren. Zur Detektion wurde eine für den 5' Tail des Uni Gap dT Primer spezifische Fluoreszenz-markierte Sonde eingesetzt. Die Aufnahme der Fluoreszenzdaten erfolgte während des 52°C Annealing/ Extensions-Schrittes. Die "3in1" Reaktion wurde mit einem Applied Biosystems 7500 Fast Real-Time PCR System (Applied Biosystems) in einem Reaktionsvolumen von 20 µl durchgeführt.

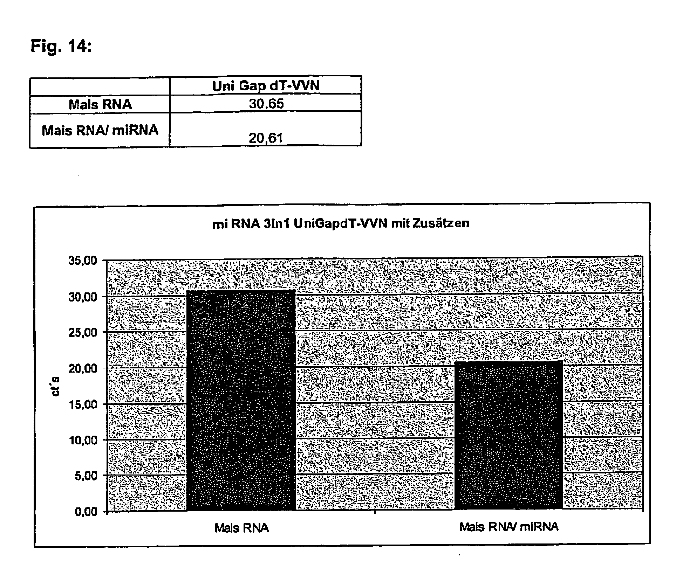

Wie in Abbildung 14 gezeigt, erlaubt die "3in1" Reaktion den spezifischen Nachweis des synthetischen 22mer RNA Oligonukleotid im Hintergrund von Mais RNA. Die Probe, welche das synthetischen 22mer RNA Oligonukleotid im Hintergrund von Mais RNA enthielt liefert einen Ct Wert von 20,61, wobei die Kontrollreaktion mit Mais RNA einen Ct Wert von 30,65 lieferte. Dieses Experiment zeigt das die "3in1" Reaktion unter den gegebenen Bedingungen technisch umsetzbar ist

### Beispiel 8:

Durchführung des erfindungsgemäßen "3in1" Verfahrens unter Verwendung eines manuellen PCR Primer "Hot-Starts", mit dem Ziel die Reaktion des gekoppelten dreistufigen Prozesses zu begünstigen. Als Kontrolle wurde die Reaktion in einem zweistufigen Verfahren in Anlehnung an die Abbildung 1B durchgeführt.
Die Reaktionen wurden mit den in Tabelle 43 angegebenen Materialien wie in Tabelle 44 angegeben zusammengesetzt.

**Tabelle 43: Materialien für die Poly A Reaktion, Reverse Transkription und PCR**

| | | |
|---|---|---|
| Poly (A) Polymerase | | Epicentre Biotechnologies; Material Nummer 400U: PAP5104 |
| QuantiTect Multiplex RT-PCR Kit | | Qiagen; Material Nummer 200 rxns: 204643 |
| Adenosine 5'-Triphosphate (rATP) | | Amersham; Material Nummer 25µmol: 27-2056-01 |
| dNTP Mix (ATGC je 10mM) | | Amersham; Material Nummer Qiagen Intern 1007430 |
| RNase Inhibitor | | Promega: Material Nummer: N2511 |
| Uni GAP dT Primer | | |
| Mais RNA | | Aus 1g gemörserten Maisblättern mit Qiagen RNeasy Mini Kit (Cat. No. 74106) Plant Protokoll, 10 facher upscale (Maxi shredder und Säule) siehe oben |
| mleu7a Oligonukleotid | | 5'-UGA GGU AGU AGG UUG UAU AGU U-3' |

| **PCR Primer:** | | |
|---|---|---|
| Lct 7short (spezifischer miRNA Primer) | 5'-GAG GTA GTA GGT TGT ATA G-3' | |
| Hum Uni Primer | 5'-AAC GAG ACG ACG ACA GAC-3' | |
| GAPDH-TM-HEX_BHQ | 5'HEX-CAA GCT TCC CGT TCT CAG CC-BHQ 3' | |

| **Zusätze:** | | |
|---|---|---|
| Poly A RNA Random N8 Primer Oligo dT 12 | Amersham Biosciences Material Nummer 27-4110-01, gelöst mit 25µg/µl in RNase freiem Wasser NNNNNNNN TTTTTTTTTTTT-3' Phosphat | |

**Tabelle 44: PAP + RT Reaktion + PCR in QuantiTect Multiplex RT-PCR Master Mix (Qiagen)**

| Reagenzien | Finale Konzentration |
|---|---|
| 2x QuantiTect Multiplex RT-PCR Master Mix | 1 x |
| rATP 10 mM | 100 µM |
| Poly A Polymerase 4U/ µl | 1 U |
| dNTP Mix (ATGC je 5mM) | 0,5 mM |
| Uni GAP dT Primer 10 µM | 0,05 µM |
| RNase Inhibitor 40 U/ µl | 10 U |
| QuantiTect Multiplex RT Mix | 0,2µl |
| RNase freies Wasser | variabel |
| Poly A 25µg/µl | 10ng/µl |
| N8 Random Primer | 0,05µM |
| Oligo dT12 | 5µM |
| mleu7a 10^9 Kopien/µl | 2 µl (2x 10⁹ Kopien) |
| Mais RNA 20 ng/µl | 2 µl (40 ng) |
| Gesamtvolumen | 20 µl |

Mit den PCR Primern aus Tabelle 45 wird ein Primer Mix hergestellt und die benötigte Menge für jeweils eine Reaktion in jeweils einen Deckel eines Optical Caps (Deckel für Real-time PCR Gefäße, Applied Biosystems; Material Nummer 4323032) pipettiert. Anschließend wurden die Deckel auf einem Heizblock bei 37°C für ca. 20 min inkubiert, bis die Flüssigkeit verdampft und somit die Primer getrocknet waren.

Nach der vollständigen Trocknung der PCR Primer im Deckel werden die Reagenzien wie in Tabelle 44 zusammenpipettiert und in Optical Tubes gegeben (Real-time PCR Gefäße, Applied Biosystems; Material Nummer 4316567), mit den vorbehandelten Optical caps verschlossen und die PCR wie in Tabelle 47 angegeben durchgeführt.
Die Reaktionen wurden in Dreifachbestimmungen getestet.

**Tabelle 45: Zusammensetzung getrockneter Oligo-Mix im PCR-Deckel**

| | Menge Oligo/ rxn | **Finale Konzentration in PCR nach dem Rücklösen** |
|---|---|---|
| Hum Uni Primer | 10 pmol | 0,5 µM |
| let 7short (spezifischer miRNA Primer) | 10 pmol | 0,5 µM |
| GAPDH-TM-HEX BHQ | 4 pmol | 0,2µM |

**Tabelle 46: Reaktionsprotokoll der "3in1" Reaktion**

| | | |
|---|---|---|
| Poly A Reaktion und Reverse Transkription | 45 min 37°C | - |
| | 15min 50°C | |
| Inkubation | 95°C 3min | |
| 8er-Strip Reaktionsgefäße kurz invertieren, um die im Deckel getrockneten Primer im Reaktionsmix zu lösen | | |
| PCR Initiale Reaktivierung | 12 min 95°C | |
| Denaturierung | 15 sec 94°C | 45x |
| Annealing/ Extension | 30 sec 52°C | |

Das Reaktionsprotokoll bestand zuerst aus Bedingungen für die kombinierte Reaktion von Poly-A Polymerase und der reversen Transkription mit dem QuantiTect Multiplex Reverse Transcriptase Mix (45 min 37°C und 15 min 50°C). Anschließend wurden die Reaktionen für 3 min auf 95°C erhitzt, mit dem Ziel die Poly-A-Polymerase und Reverse Transkriptase Enzyme zu inaktivieren. Danach wurden die PCR Tubes kurz aus dem Gerät genommen und invertiert, mit dem Ziel die in den Deckeln vorliegenden getrockneten Primer rückzulösen und für die folgende PCR Reaktion verfügbar zu machen. Darauf folgte eine Inkubation für 12 min bei 95°C, um die im QuantiTect Multiplex RT-PCR Master Mix enthaltenen HotStarTaq DNA Polymerase zu aktivieren. Es wurde hier eine um 3 min kürzere Reaktivierung gewählt, da die Reaktionsmixe bereits vorher zur Inaktivierung der Poly-A-Polymerase und Reverse Transkriptase Enzyme für 3 min auf 95°C erhitzt wurde. Es folgten 45 PCR Zyklen mit 15 sec bei 94°C und 30 sec bei 52°C (siehe Tabelle 47), um die generierte let7a-cDNA in einer real-time PCR zu amplifizieren. Zur Detektion wurde eine für den 5' Tail des Uni Gap dT Primer spezifische Fluoreszenz-markierte Sonde eingesetzt. Die Aufnahme der Fluoreszenzdaten erfolgte während des 52°C Annealing/ Extensions-Schrittes. Die "3in1" Reaktion wurde mit einem Applied Biosystems 7500 Real-Time PCR System (Applied Biosystems) in einem Reaktionsvolumen von 20 µl durchgeführt

### FIGUREN

**Fig. 1** zeigt einen Vergleich zwischen den einstufigen Verfahren gemäß vorliegender Erfindung (B) und dem Zweistufenprozess wie er im Stand der Technik bekannt ist (A). Pol A Polymerase: Enzym mit Polyadenyleringsaktivität im sinne der Erfindung; Reverse Transkriptase: Enzym mit Reverser Transkriptaseaktivität im sinne der Erfindung; rATP: Ribonukleotid, hier beispielhaft Adenosin-5'-Triphosphat; dNTPs: Desoxyribonukleotide; Oligo dT Tail Primer: Anker Oligonukleotid mit verschiedenen möglichen Ausführungsformen im Sinne der Erfindung; Uni GAP dT Primer: spezielle Ausführungsform des Anker Oligonukleotids; Tail: 5' Tail als optionaler Teil des Anker Oligonukleotids; w: definiert die erfindungsgemäße Länge des durch die Polyadenylierungsaktivität angefügten homopolymer Tails (größer 10-20 Basen); x,y: definiert die erfindungsgemäße Art und Länge der 3' Ankersequenz des erfindungsgemäßen Anker Oligonukleotids; z: definiert die Länge des homopolymer Anteils des erfindungsgemäßen Anker Oligonukleotids.
**Fig. 2** zeigt eine grafische Darstellung der Ct Werte aus Tabelle 6: Bedingung a) enthielt jeweils Template, bei Bedingung b) wurde anstatt Template nur H₂O zugesetzt (H₂O in PAP Reaktion). Bei b) wurde kein Signal bis zu PCR Zyklus 40 (maximale Zahl der durchgeführten Zyklen) erhalten, daher Angabe "Kein Ct".
**Fig. 3** zeigt eine Agarosegel-Analyse der Real-time PCR Produkte aus Beispiel 1. M: 100 bp Ladder (Invitrogen, Katalog-Nr. 15628-050). 2% Agarose, gefärbt mit Ethidiumbromid in TAE als Laufpuffer. Ladeschema: Spur 1: Marker; dann wurden jeweils die Dreifachbestimmungen nebeneinander aufgetragen; 1a, 1b, 2a, 2b, 3a, 3b, 4a, 4b.

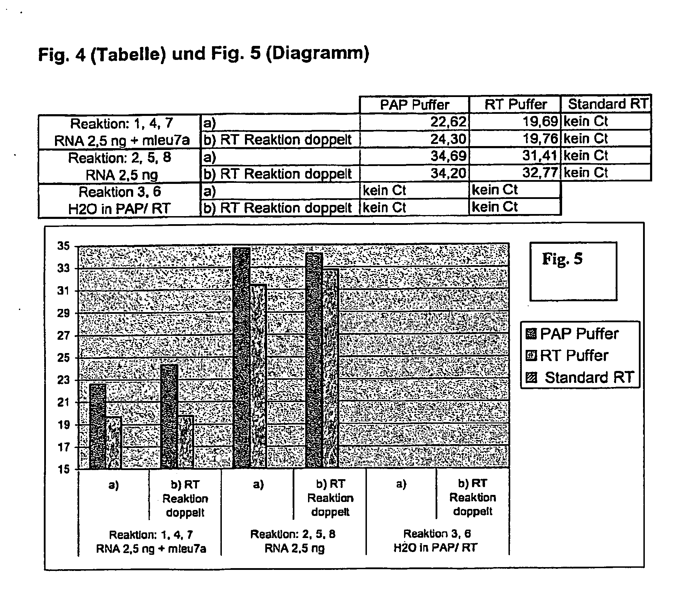

**Fig. 4** zeigt eine tabellarische Darstellung von Ct Werten, die durch real-time PCR Analyse der Reaktionsprodukte der in Tabelle 8 beschriebenen Ansätzen erhalten wurden. Bei 3 und 6 wurde kein Signal bis zu PCR Zyklus 40 (maximale Zahl der durchgeführten Zyklen) erhalten, daher Angabe "kein Ct".
**Fig. 5** zeigt eine grafische Darstellung von Ct Werten, die durch real-time PCR Analyse der Reaktionsprodukte der in Tabelle 8 beschriebenen Ansätzen erhalten wurden.
**Fig. 6** zeigt eine tabellarische Darstellung von Ct Mittelwerten, die durch real-time PCR Analyse der Reaktionsprodukte der in Tabelle 18 beschriebenen Ansätzen b) und e) erhalten wurden.
   Bei 2, 4 und 5 mit Standard RT wurde ein Signal frühestens erst nach Zyklus 38 detektiert, bzw. bei 2) kein Signal, daher Angabe "kein Ct".
**Fig. 7** zeigt eine tabellarische Darstellung der real-time PCR Ergebnisse der Ansätze b) und d) aus Tabelle 18, sowie der Kontrollen mit nur einem Primer aus Tabelle 19 Ansätze a) -d). Es wurde kein Signal bis zu PCR Zyklus 40 (maximale Zahl der durchgeführten Zyklen) erhalten, daher Angabe "kein Ct".
**Fig. 8** zeigt eine Agarosegel-Analyse der Real-time PCR Produkte aus Beispiel 3. M: 100 bp Ladder (Invitrogen, Katalog-Nr. 15628-050). 2% Agarose, gefärbt mit Ethidiumbromid in TAE als Laufpuffer.
**Fig. 9** zeigt eine tabellarische Darstellung von Ct Mittelwerten, die durch real-time PCR Analyse der Reaktionsprodukte der in Tabelle 30 beschriebenen Ansätzen 1 a) b) und 2 a) b) erhalten wurden.
   **Unterer Teil:** Grafische Darstellung von Ct Mittelwerten, die durch real-time PCR Analyse der Reaktionsprodukte der in Tabelle 30 beschriebenen Ansätzen erhalten wurden.

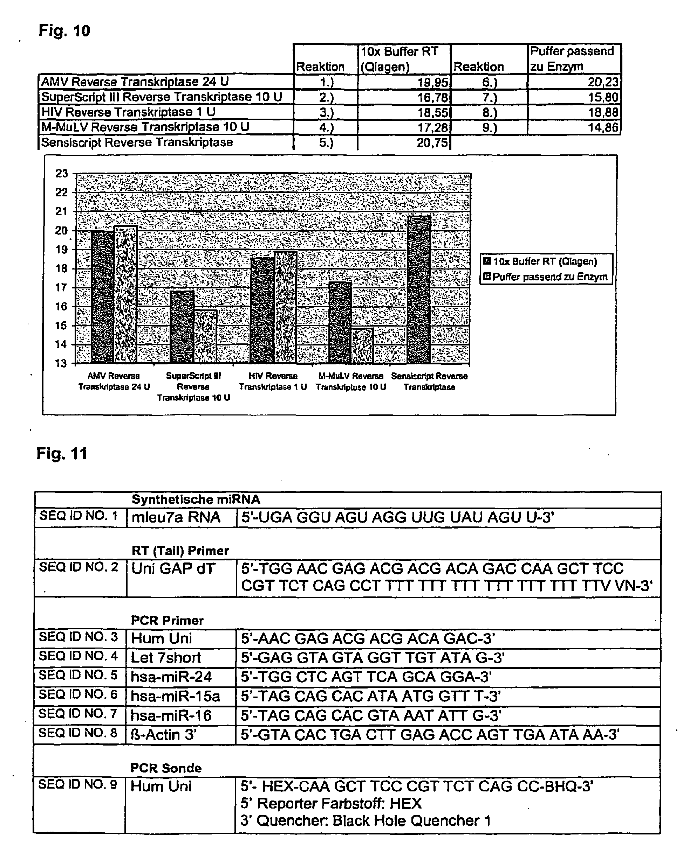

**Fig. 10** zeigt eine tabellarische Darstellung von Ct Mittelwerten, die durch real-time PCR Analyse der Reaktionsprodukte der in Tabelle 36 1-5 und in Tabelle 37 6-9 beschriebenen Ansätzen erhalten wurden.
   **Unterer Teil:** Grafische Darstellung von Ct Mittelwerten, die durch real-time PCR Analyse der Reaktionsprodukte der in Tabelle 36 1-5 und in Tabelle 37 6-9 beschriebenen Ansätzen erhalten wurden.
**Fig. 11** zeigt eine Liste der verwendeten Nukleinsäuresequenzen.
**Fig. 12** zeigt erfindungsgemäße Anker Oligonukleotide.

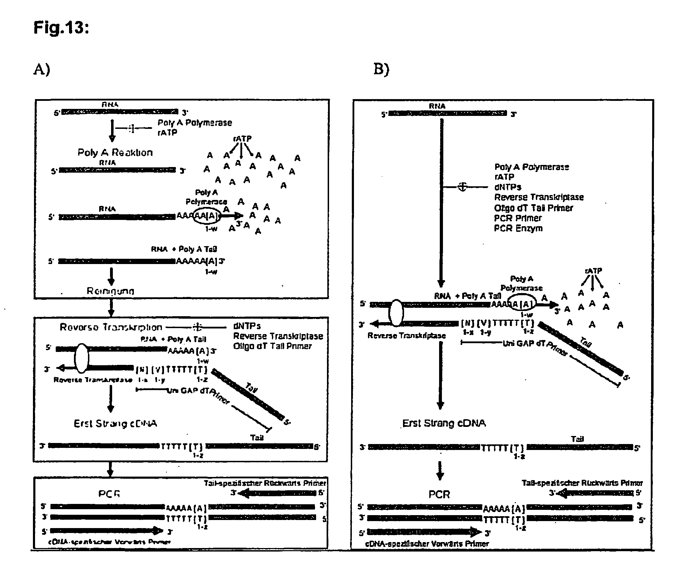

**Fig. 13** zeigt einen Vergleich zwischen den einstufigen "3in1" Verfahren gemäß vorliegender Erfindung (B) und dem Dreistufenprozess wie er im Stand der Technik bekannt ist (A). Pol A Polymerase: Enzym mit Polyadenyleringsaktivität im Sinne der Erfindung;
   Reverse Transkriptase: Enzym mit Reverser Transkriptaseaktivität im Sinne der Erfindung; rATP: Ribonukleotid, hier beispielhaft Adenosin-5'-Triphosphat; dNTPs: Desoxyribonukleotide; Oligo dT Tail Primer: Anker Oligonukleotid mit verschiedenen möglichen Ausführungsformen im Sinne der Erfindung; Uni GAP dT Primer: spezielle Ausführungsform des Anker Oligonukleotids; Tail: 5' Tail als optionaler Teil des Anker Oligonukleotids; w: definiert die erfindungsgemäße Länge des durch die Polyadenylierungsaktivität angefügten homopolymer Tails (größer 10-20 Basen); x,y: definiert die erfindungsgemäße Art und Länge der 3' Ankersequenz des erfindungsgemäßen Anker Oligonukleotids; z: definiert die Länge des homopolymer Anteils des erfindungsgemäßen Anker Oligonukleotids. PCR Primer: mindestens ein Oligonukleotid zum spezifischen Nachweis von cDNA Spezies, optional mindestens eine Sonde; PCR Enzym: Enzymatische Aktivität welche den spezifischen Nachweis von in der Probe enthaltenen cDNA Spezies erlaubt.
**Fig. 14** zeigt eine tabellarische Darstellung von Ct Mittelwerten einer "3in1 Reaktion, d.h. der kombinierten Poly-(A)-Polymerase Reaktion, Reverse Transkription und real-time PCR Analyse gekoppelt in einem Reaktionsgefäß, nach Reaktionsansatz aus Beispiel 7 entsprechend Tabelle 41 und dem Reaktionsansatz aus Tabelle 42.
   **Unterer Teil:** Grafische Darstellung von Ct Mittelwerten einer "3in1 Reaktion, d.h. der kombinierten Poly-(A)-Polymerase Reaktion, Reverse Transkription und real-time PCR Analyse gekoppelt in einem Reaktionsgefäß, nach Reaktionsansatz aus Beispiel 7 entsprechend Tabelle 41 und dem Reaktionsansatz aus Tabelle 42.

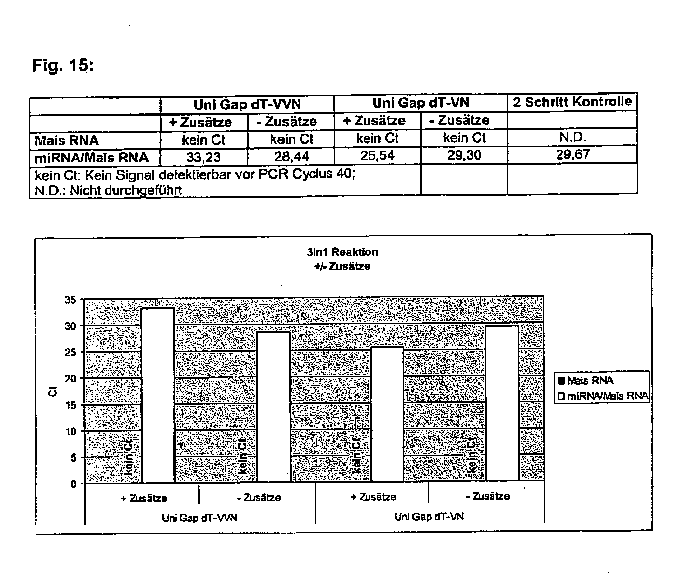

**Fig. 15** zeigt eine tabellarische Darstellung von Ct Mittelwerten einer "3in1 Reaktion, d.h. der kombinierten Poly-(A)-Polymerase Reaktion, Reverse Transkription und real-time PCR Analyse gekoppelt in einem Reaktionsgefäß, nach Reaktionsansatz Reaktionsansatz aus Beispiel 8 entsprechend Tabelle 44 und dem Reaktionsansatz aus Tabelle 46.
   **Unterer Teil:** Grafische Darstellung von Ct Mittelwerten einer "3in1 Reaktion, d.h. der kombinierten Poly-(A)-Polymerase Reaktion, Reverse Transkription und real-time PCR Analyse gekoppelt in einem Reaktionsgefäß.

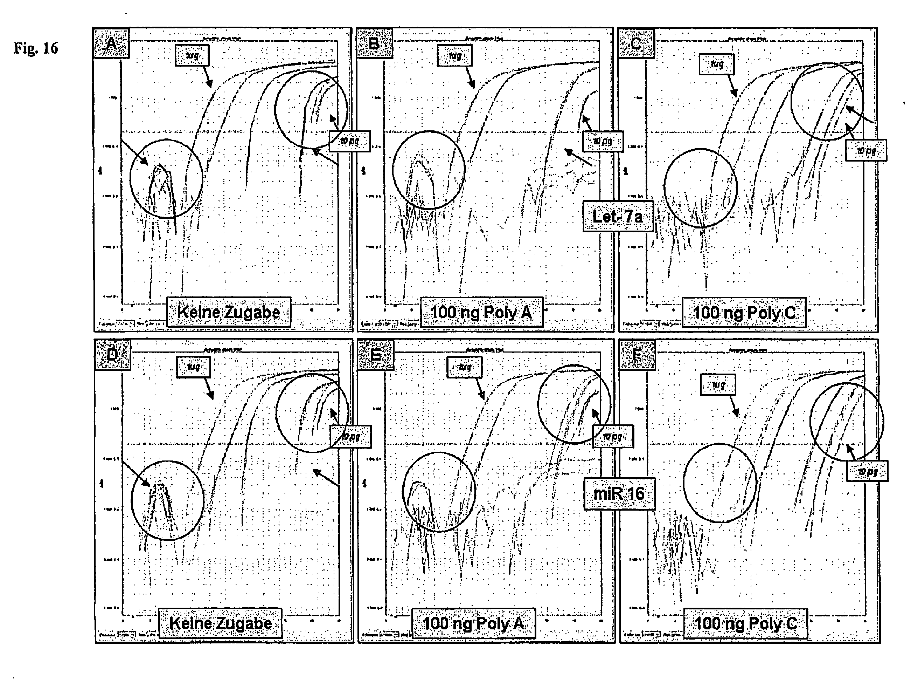

**Fig. 16** zeigt verschiedene Mengen (10 pg bis 1 µg) von miRNAeasy RNA, die unter Verwendung von miScript in der Gegenwart oder Abwesenheit von 100 ng Poly-(A) oder Poly-(C) revers transkribiert wurden. Die dadurch erzeugte cDNA wurde in einer real-time PCR verwendet; in diesem Fall wurden miR-16 und let-7a getestet.

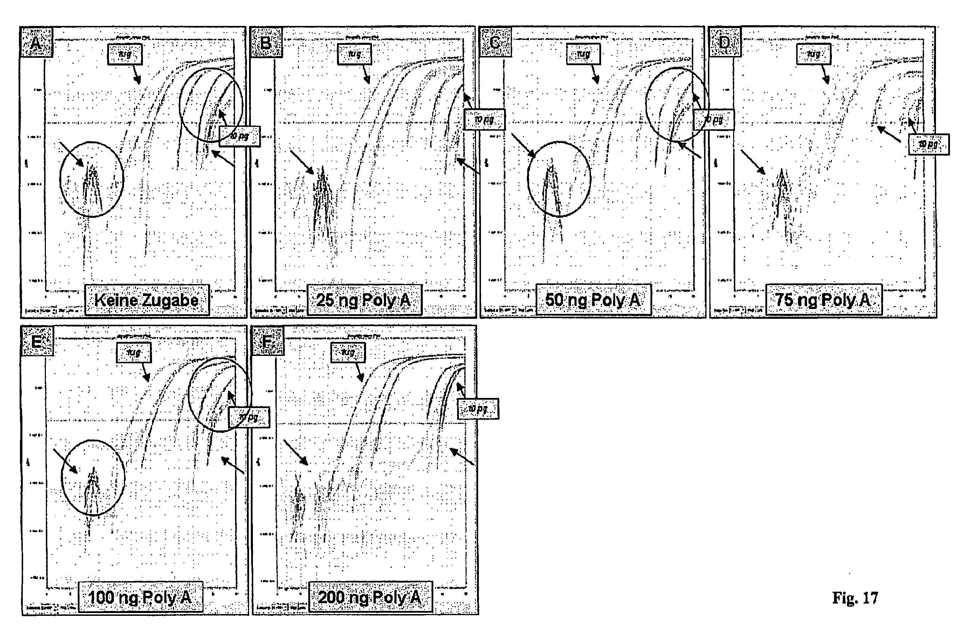

**Fig. 17** zeigt verschiedene Mengen (10 pg bis 1 µg) von miRNAeasy RNA, die unter Verwendung von miScript in der Gegenwart oder Abwesenheit von verschiedenen Mengen Poly-(A) revers transkribiert wurden. Die dadurch erzeugte cDNA wurde in einer real-time PCR verwendet; in diesem Fall wurde miR-16 getestet.

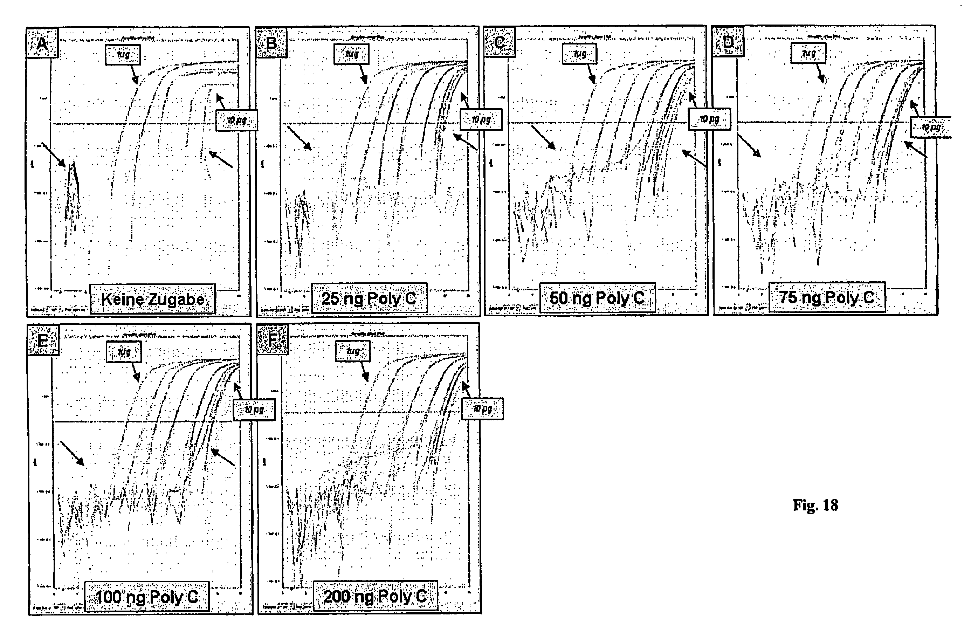

**Fig. 18** zeigt verschiedene Mengen (10 pg bis 1 µg) von miRN Aeasy RNA, die unter Verwendung von miScript in der Gegenwart oder Abwesenheit von verschiedenen Mengen Poly-(C) revers transkribiert wurden. Die dadurch erzeugte cDNA wurde in einer real-time PCR verwendet; in diesem Fall wurde miR-16 getestet.

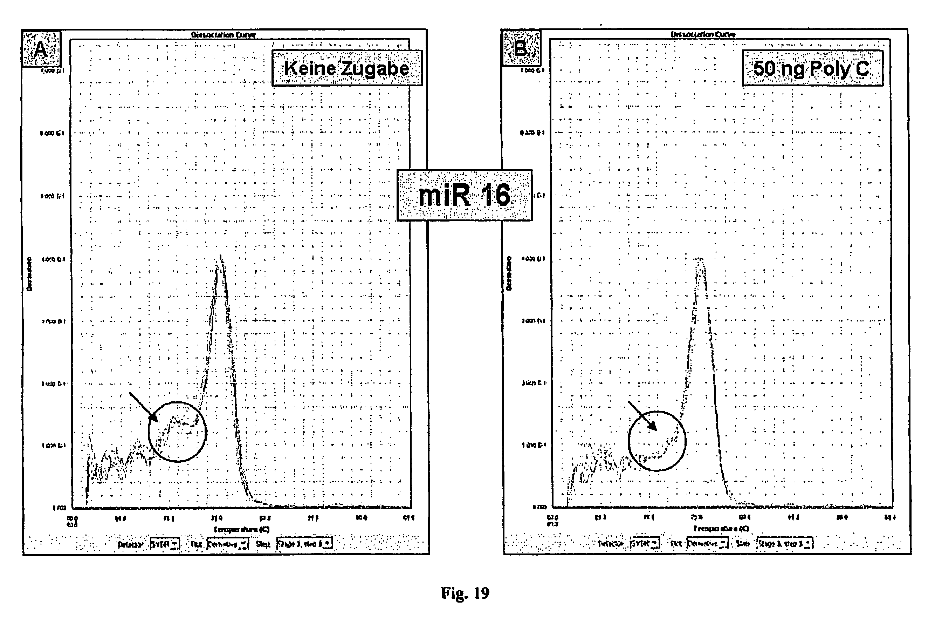

**Fig. 19** zeigt die Verwendung von 10 pg miRNeasy RNA, die in Gegenwart oder Abwesenheit von 50 ng Poly-(C) unter Verwendung des miScript RT Kits revers transkribiert wurde.
Die dadurch erzeugte cDNA wurde in einer real-time PCR verwendet um GAPDH nachzuweisen.

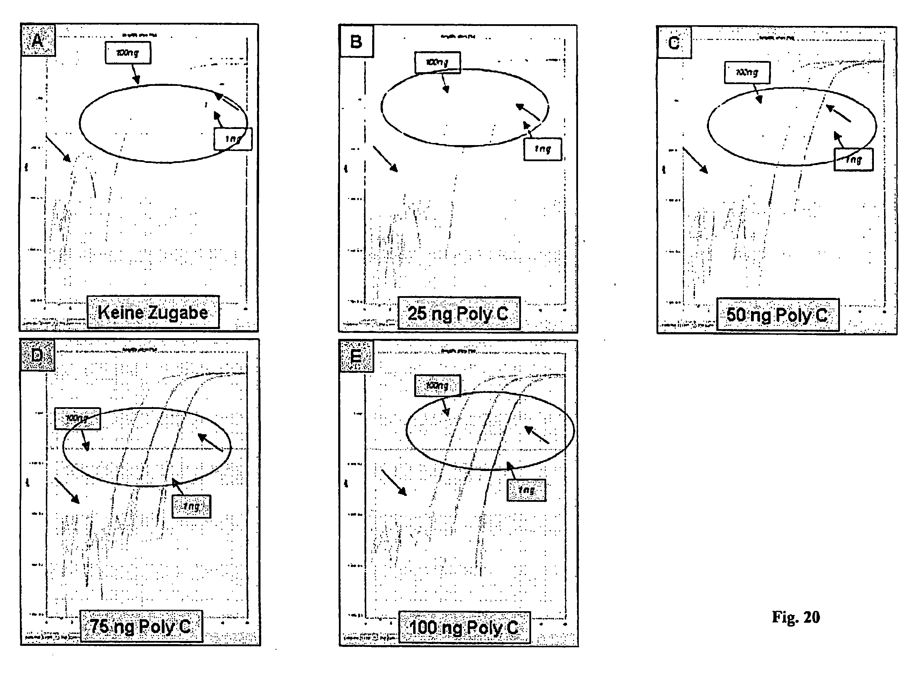

**Fig. 20** zeigt verschiedene Mengen (1 - 100 ng) miRNeasy RNA, die unter Verwendung von miScript in der Gegenwart oder Abwesenheit von verschiedenen Mengen Poly-(C) revers transkribiert wurden. Die dadurch erzeugte cDNA wurde in einer real-time PCR verwendet; in diesem Fall um GADPH zu testen.

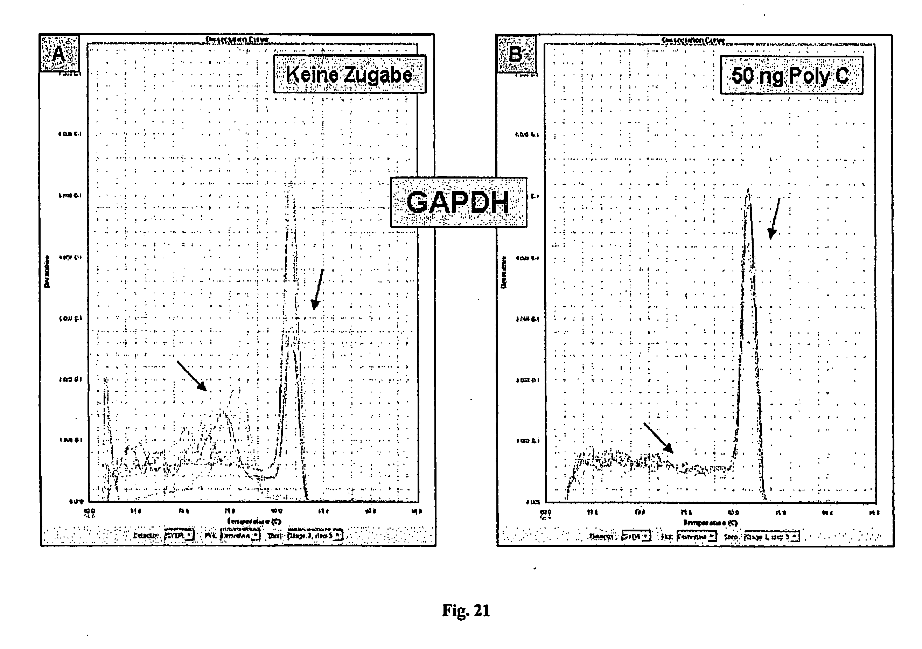

**Fig. 21** zeigt die Verwendung von 10 und 100 pg miRNeasy RNA, die in der Gegenwart oder Abwesenheit von 50 ng Poly-(C) und unter Verwendung des miScript RT Kits revers transkribiert wurde. Die dadurch erzeugte cDNA wurde in einer real-time PCR getestet um GAPDH nachzuweisen.

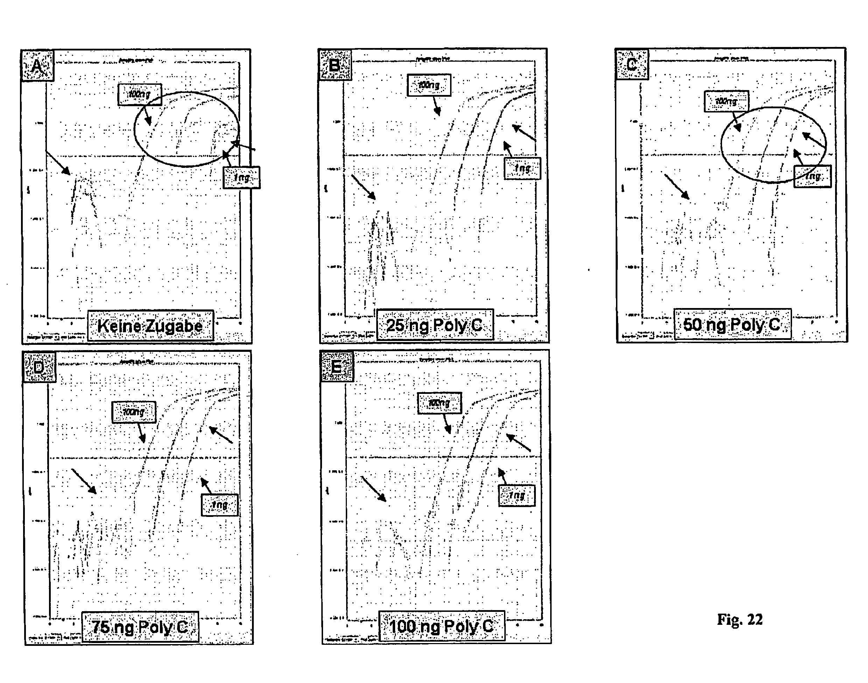

**Fig. 22** zeigt verschiedene Mengen (1 - 100 ng) miRNeasy RNA, die unter Verwendung von miScript in der Gegenwart oder Abwesenheit von verschiedenen Mengen Poly-(C) revers transkribiert wurden. Die dadurch erzeugte cDNA wurde in einer real-time PCR verwendet; in diesem Fall um CDC2 zu testen.

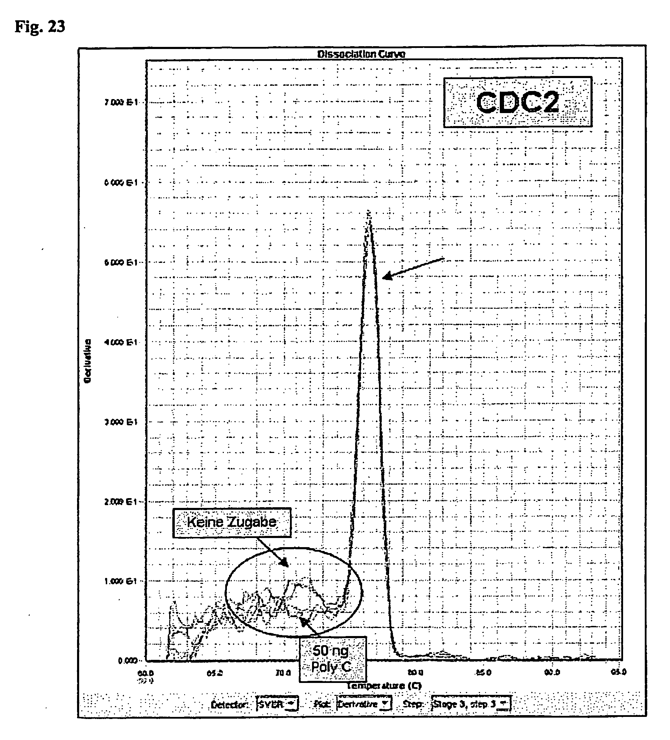

**Fig. 23** zeigt die Verwendung von 1 ng miRNeasy RNA, die in der Gegenwart oder Abwesenheit von 50 ng Poly-(C) und unter Verwendung des miScript RT Kits revers transkribiert wurde. Die dadurch erzeugte cDNA wurde in einer real-time PCR getestet um CDC2 nachzuweisen.

### SEQUENCE LISTING

<110> Qiagen GmbH
   Qiagen GmbH
<120> Verfahren zur Synthese einer cDNA in einer Probe in einer enzymatischen Reaktion
<130> M3362 PCT BLN
<160> 19
<170> PatentIn version 3.3
<210> 1
   <211> 22
   <212> RNA
   <213> Artificial
<220>
   <223> synthetisch
<400> 1
   ugagguagua gguuguauag uu 22
<210> 2
   <211> 65
   <212> DNA
   <213> Artificial
<220>
   <223> synthetisch
<220>
   <221> misc_feature
   <222> (65)..(65)
   <223> n is a, c, g, t or u
<400> 2
   tggaacgaga cgacgacaga ccaagcttcc cgttctcagc cttttttttt tttttttttt 60
   ttvvn 65
<210> 3
   <211> 18
   <212> DNA
   <213> Artificial
<220>
   <223> Sythetisch
<400> 3
   aacgagacga cgacagac 18
<210> 4
   <211> 19
   <212> DNA
   <213> Artificial
<220>
   <223> Sythetisch
<400> 4
   gaggtagtag gttgtatag 19
<210> 5
   <211> 18
   <212> DNA
   <213> Artificial
<220>
   <223> Synthetisch
<400> 5
   tggctcagtt cagcagga 18
<210> 6
   <211> 19
   <212> DNA
   <213> Artificial
<220>
   <223> Sythetisch
<400> 6
   tagcagcaca taatggttt 19
<210> 7
   <211> 19
   <212> DNA
   <213> Artificial
<220>
   <223> Sythetisch
<400> 7
   tagcagcacg taaatattg 19
<210> 8
   <211> 29
   <212> DNA
   <213> Artificial
<220>
   <223> Sythetisch
<400> 8
   gtacactgac ttgagaccag ttgaataaa 29
<210> 9
   <211> 20
   <212> DNA
   <213> Artificial
<220>
   <223> Synthetisch
<400> 9
   caagcttccc gttctcagcc 20
<210> 10
   <211> 53
   <212> DNA
   <213> Artificial
<220>
   <223> Synthetisch - poly T zwischen 10 und 30
<220>
   <221> misc_feature
   <222> (53)..(53)
   <223> n is a, c, g, or t
<400> 10
   tggaacgaga cgacgacaga ccaagctccc gttctcagcc tttttttttt vvn 53
<210> 11
   <211> 30
   <212> DNA
   <213> Artificial
<220>
   <223> Synthetisch - poly T zwischen 10 und 30
<220>
   <221> misc_feature
   <222> (30)..(30)
   <223> n is a, c, g, or t
<400> 11
   aacgagacga cgacagactt ttttttttvn 30
<210> 12
   <211> 29
   <212> DNA
   <213> Artificial
<220>
   <223> Synthetisch - poly T zwischen 10 und 30
<400> 12
   aacgagacga cgacagactt ttttttttv 29
<210> 13
   <211> 29
   <212> DNA
   <213> Artificial
<220>
   <223> Synthetisch - poly T zwischen 10 und 30
<220>
   <221> misc_feature
   <222> (29)..(29)
   <223> n is a, c, g, or t
<400> 13
   aacgagacga cgacagactt ttttttttn 29
<210> 14
   <211> 30
   <212> DNA
   <213> Artificial
<220>
   <223> Synthetisch poly T zwischen 10 und 30
<220>
   <221> misc_feature
   <222> (29)..(30)
   <223> n is a, c, g, or t
<400> 14
   aacgagacga cgacagactt ttttttttnn 30
<210> 15
   <211> 31
   <212> DNA
   <213> Artificial
<220>
   <223> Synthetisch poly T zwischen 10 und 30
<220>
   <221> misc_feature
   <222> (30)..(31)
   <223> n is a, c, g, or t
<400> 15
   aacgagacga cgacagactt ttttttttvn n 31
<210> 16
   <211> 32
   <212> DNA
   <213> Artificial
<220>
   <223> Synthetisch poly T zwischen 10 und 30
<220>
   <221> misc_feature
   <222> (30)..(32)
   <223> n is a, c, g, or t
<400> 16
   aacgagacga cgacagactt ttttttttvn nn 32
<210> 17
   <211> 31
   <212> DNA
   <213> Artificial
<220>
   <223> Synthetisch poly T zwischen 10 und 30
<220>
   <221> misc_feature
   <222> (29)..(31)
   <223> n is a, c, g, or t
<400> 17
   aacgagacga cgacagactt ttttttttnn n 31
<210> 18
   <211> 52
   <212> DNA
   <213> Artificial
<220>
   <223> Synthetisch poly T zwischen 10 und 30
<220>
   <221> misc_feature
   <222> (52)..(52)
   <223> n is a, c, g, or t
<400> 18
   tggaacgaga cgacgacaga ccaagcttcc cgtctcagcc tttttttttt vn 52
<210> 19
   <211> 53
   <212> DNA
   <213> Artificial
<220>
   <223> Synthetisch poly T zwischen 10 und 30
<220>
   <221> misc_feature
   <222> (52)..(53)
   <223> n is a, c, g, or t
<400> 19
   tggaacgaga cgacgacaga ccaagcttcc cgtctcagcc tttttttttt vnn 53

## Patentansprüche

1. Verfahren zur Synthese einer cDNA in einer Probe in einer enzymatischen Reaktion, wobei das Verfahren folgende Schritte umfasst:
a. Gleichzeitige Bereitstellung eines ersten Enzyms mit Polyadenylierungsaktivität, eines zweiten Enzyms mit reverser Transkriptaseaktivität, eines Puffers, mindestens eines Ribonukleotids, mindestens eines Desoxyribonukleotids, eines Anker Oligonukleotids umfassend eine Homopolymersequenz Poly(T),
b. Zugabe einer Probe umfassend eine Ribonukleinsäure und
c. Inkubation der Agenzien der Schritte a) und b) bei einem oder mehreren Temperaturschritten, welche so gewählt sind, dass das erste und das zweite Enzym Aktivität zeigen.

2. Verfahren nach Anspruch 1 wobei die Probe eine Ribonukleinsäure umfasst, die ausgewählt ist aus der Gruppe umfassend prokaryontische RNA, eukaryontische RNA, Virale RNA, Archae-RNA, miRNA, snoRNA, mRNA, tRNA, nicht-polyadenylierte RNA, und rRNA sowie Gemische davon.

3. Verfahren nach Anspruch 2, wobei das Anker Oligonukleotid eine Länge zwischen 6 und 150 Nukleotiden hat, und optional am 3'-Ende eine Ankersequenz aufweist.

4. Verfahren nach Anspruch 1 bis 3, wobei das Ribonukleotid ausgewählt sein kann aus der Gruppe umfassend, Adenosin-5'-Triphosphat, Thymin-5'-Triphosphat, Cytosin-5'-Triphosphat, Guanin-5'-Triphosphat, Uracil-5'-Triphosphat, ein Ribonukleotid mit einem Basenanalogon, und wobei das Ribonukleotid optional modifiziert oder markiert sein können.

5. Verfahren nach Anspruch 1 bis 4, wobei das Desoxyribonukleotid ausgewählt sein kann aus der Gruppe umfassend, Desoxyadenosin-5'-Triphosphat (dATP), Desoxythymin-5'-Triphosphat (dTTP), Desoxycytosin-5'-Triphosphat (dCTP), Desoxyguanin-5'-Triphosphat (dGTP), Desoxyuracil-5'-Triphosphat (dUTP) und wobei das Desoxyribonucleotid optional modifiziert oder markiert sein kann.

6. Verfahren nach Anspruch 5, wobei die Konzentration eines Desoxyribonukleotids wenigstens 0,01 mM in der Reaktion beträgt und höchstens 10 mM in der Reaktion beträgt.

7. Verfahren nach Anspruch 6, wobei die Desoxyribonukleotide dATP, dCTP, dGTP und dTTP in einer Konzentration von 0,2 mM bis 2 mM vorliegen.

8. Verfahren nach einem der Ansprüche 1 bis 7, wobei der Puffer einen pH von 6 bis 10 hat und Mg2+ Ionen umfasst.

9. Verfahren nach einem der obigen Ansprüche, wobei das Enzym mit reverser Transkriptaseaktivität ausgewählt ist aus der Gruppe umfassend, Enzyme aus Viren, Bakterien, Archae-Bakterien, Eukaryonten und Enzyme, insbesondere aus thermostabilen Organismen und Enzyme, die eine solche Funktion erst durch Veränderung ihrer Gensequenz Mutagenese oder durch entsprechende Pufferbedingungen erhalten.

10. Verfahren nach Anspruch 9, wobei das Enzym mit reverser Transkriptaseaktivität ausgewählt ist aus der Gruppe umfassend, HIV reverse Transkriptase, M-MLV reverse Transkriptase, EAIV reverse Transkriptase, AMV reverse Transkriptase, Thermus thermophilus DNA Polymerase 1, M-MLV RNAse H-(Superscript, Superscript **II,** Superscript III), Monstersript (Epicentre), Omniscript, Sensiscript Reverse Transkriptase (Qiagen), ThermoScript und Thermo-X (beide Invitrogen), AccuScript reverse Transcriptase (Stratagene).

11. Verfahren nach den Ansprüchen 1 bis 10; wobei die Reaktion zusätzlich einen Temperaturschritt bei einer höheren Temperatur von etwa 65°C bis 95°C umfasst.

12. Verfahren nach den Ansprüchen 1 bis 11, wobei die im Verfahren generierte cDNA anschließend mit einer Polymerasenkettenreaktion amplifiziert wird und die Reaktion Randomprimer umfasst und/oder spezifische Primer und/oder optional eine oder mehrere Sonden umfasst.

13. Reaktionsgemisch umfassend ein erstes Enzym mit Polyadenylierungsaktivität, ein zweites Enzym mit reverser Transkriptaseaktivität, Kompetitor Poly-(C)-Polynukleotide, optional einen Puffer, optional mindestens ein Ribonukleotid, optional mindestens ein Desoxyribonukleotid und optional ein Anker Oligonukleotid, optional Random Primer sowie optional ein Enzym mit DNA-Syntheseaktivität.

14. Kit umfassend ein Reaktionsgemisch nach Anspruch 13.

15. Verwendung eines Reaktionsgemisches umfassend
ein erstes Enzym mit Polyadenylierungsaktivität, ein zweites Enzym mit reverser Transkriptaseaktivität, optional ein Anker Oligonukleotid, optional Poly-(C)-Polynukleotide, optional einen Puffer, optional mindestens ein Ribonukleotid, optional mindestens ein Desoxyribonukleotid und , optional Random Primer, sowie optional ein Enzym mit DNA-Syntheseaktivität
in einem Verfahren nach den Ansprüchen 1 bis 12

## Claims

1. Method for synthesis of a cDNA in a sample with an enzymatic reaction, wherein the method comprises the following steps:
a) simultaneously providing a first enzyme with polyadenylation activity, a second enzyme with reverse transcriptase activity, a buffer, at least one ribonucleotide, at least one deoxyribonucleotide, an anchor oligonucleotide comprising a homopolymer sequence poly(T),
b) adding a sample comprising a ribonucleic acid and
c) incubating the agents of step a) and b) under one or more temperature steps, which are selected such that the first and the second enzyme show activity.

2. Method according to claim 1, wherein the sample comprises a ribonucleic acid selected from the group comprising prokaryotic RNA, eukaryote RNA, viral RNA, archae-RNA, miRNA, snoRNA, mRNA, tRNA, non-polyadenylated RNA and rRNA as well as mixtures thereof.

3. Method according to claim 2, wherein the anchor oligonucleotide has a length between 6 and 150 nucleotides and, optionally, has an anchor sequence at the 3'-end.

4. Method according to claims 1 to 3, wherein the ribonucleotide may be selected from the group comprising adenosine-5'-triphosphate, thymine-5'-triphosphate, cytosine-5'-triphosphate; guanine-5'-triphosphate, uracil-5'-triphosphate, a ribonucleotide with a base analog, and wherein, optionally, the ribonucleotide may be modified or labeled.

5. Method according to claims 1 to 4, wherein the deoxyribonucleotide may be selected from the group comprising desoxyadenosine-5'-triphosphate (dATP), desoxythymine-5'-triphosphate (dTTP), desoxycytosine-5'-triphosphate (dCTP), desoxyguanine-5'-triphosphate (dGTP), desoxyuracile-5'-triphosphate (dUTP) and wherein, optionally, the deoxyribonucleotide may be modified or labeled.

6. Method according to claim 5, wherein the concentration of a deoxyribonucleotide in the reaction is at least 0.01 mM and 10 mM at most.

7. Method according to claim 6, wherein the deoxyribonucleotides dATP, dTTP, dGTP, dCTP are present in a concentration from 0.2 mM to 2 mM.

8. Method according to claims 1 to 7, wherein the buffer has a pH from 6 to 10 and comprises Mg²⁺ ions.

9. Method according to one of the claims above, wherein the enzyme with reverse transcriptase activity is selected from the group comprising enzymes from viruses, bacteria, archaea bacteria, eukaryotes and enzymes, particularly, from thermostable organism, and enzymes that acquire such a function by modifying its gene sequence, mutagenesis or through adequate buffer conditions.

10. Method according to claim 9, wherein the enzyme with reverse transcriptase activity is selected from the group comprising HIV reverse transcriptase, M-MLV reverse transcriptase, EAIV reverse transcriptase, AMV reverse transcriptase, Thermus thermophiles DNA polymerase I, M-MLV RNAse H-(Superscript, Superscript II, Superscript III), Monsterscript (Epicentre), Omniscript, Sensiscript reverse transcriptase (Qiagen), ThermoScript and Thermo-X (both Invitrogen), AccuScript reverse transcriptase (Stratagene).

11. Method according to claims 1 to 10, wherein the reaction additionally comprises a temperature step at a higher temperature of about 65°C to 95°C.

12. Method according to claims 1 to 11, wherein the cDNA generated in the method is subsequently amplified by a polymerase chain reaction, and wherein the reaction comprises random primers and/or specific primers and/or, optionally, one more probes.

13. Reaction mixture comprising a first enzyme with polyadenylation activity, a second enzyme with reverse transcriptase activity, competitor poly-(C)-polynucleotides, optionally a buffer, optionally at least one ribonucleotide, optionally at least one deoxyribonucleotide and optionally an anchor oligonucleotide, optionally random primers and optionally an enzyme with DNA synthesis activity.

14. Kit comprising a reaction mixture according to claim 13.

15. Use of a reaction mixture in a method according to claims 1 to 12 comprising a first enzyme with polyadenylation activity, a second enzyme with reverse transcriptase activity, optionally an anchor oligonucleotide, optionally poly-(C)-polynucleotides, optionally a buffer, optionally at least one ribonucleotide, optionally at least one deoxyribonucleotide, optionally random primers, and optionally an enzyme with DNA-synthesis activity.

## Revendications

1. Procédé de synthèse d'un ADNc dans un échantillon dans une réaction enzymatique, où le procédé comprend les étapes suivantes :
a. fourniture simultanée d'une première enzyme à activité de polyadénylation, d'une seconde enzyme à activité de transcriptase inverse, d'un tampon, d'au moins un ribonucléotide, d'au moins un désoxyribonucléotide, d'un oligonucléotide d'ancrage comprenant une séquence homopolymère poly(T),
b. addition d'un échantillon comprenant un acide ribonucléique et
c. incubation des agents des étapes a) et b) dans une ou plusieurs étapes de température, qui sont choisies de telle manière que la première et la seconde enzyme présentent une activité.

2. Procédé selon la revendication 1 où l'échantillon comprend un acide ribonucléique qui est choisi dans le groupe comprenant les ARN procaryotes, les ARN eucaryotes, les ARN viraux, les ARN d'archae, les ARNmi, les ARMsno, les ARNm, les ARNt, les ARN non polyadénylés et les ARNr ainsi que leurs mélanges.

3. Procédé selon la revendication 2 où l'oligonucléotide d'ancrage a une longueur entre 6 et 150 nucléotides, et comprend éventuellement à l'extrémité 3' une séquence d'ancrage.

4. Procédé selon les revendications 1 à 3 où le ribonucléotide peut être choisi dans le groupe comprenant l'adénosine-5'-triphosphate, le thymine-5'-triphosphate, le cytosine-5'-triphosphate, le guanine-5'-triphosphate, l'uracile-5'-triphosphate, un ribonucléotide avec un analogue de base, et où le ribonucléotide peut éventuellement être modifié ou marqué.

5. Procédé selon les revendications 1 à 4 où le désoxyribonucléotide peut être choisi dans le groupe comprenant le désoxyadénosine-5'-triphosphate (dATP), le désoxythymine-5'-triphosphate (dTTP), le désoxycytosine-5'-triphosphate (dCTP), le désoxyguanine-5'-triphosphate (dGTP), le désoxyuracile-5'-triphosphate (dUTP) et où le désoxyribonucléotide peut éventuellement être modifié ou marqué.

6. Procédé selon la revendication 5 où la concentration d'un désoxyribonucléotide est d'au moins 0,01 mM dans la réaction et d'au plus 10 mM dans la réaction.

7. Procédé selon la revendication 6 où les désoxyribonucléotides dATP, dCTP, dGTP et dTTP sont présents en une concentration de 0,2 mM à 2 mM.

8. Procédé selon l'une des revendications 1 à 7 où le tampon a un pH de 6 à 10 et comprend des ions Mg2+.

9. Procédé selon l'une des revendications précédentes où l'enzyme à activité de transcriptase inverse est choisie dans le groupe comprenant les enzymes de virus, de bactéries, d'archaebactéries, d'eucaryotes et les enzymes en particulier d'organismes thermostables et les enzymes qui n'acquièrent une telle fonction que par modification de leur séquence génique, mutagenèse ou par des conditions de tampon correspondantes.

10. Procédé selon la revendication 9 où l'enzyme à activité de transcriptase inverse est choisie dans le groupe comprenant la transcriptase inverse de VIH, la transcriptase inverse de M-MLV, la transcriptase inverse d'EAIV, la transcriptase inverse d'AMV, l'ADN polymérase I de Thermus thermophilus, la RNAse H-(Superscript, Superscript II, Superscript III) de M-MLV, les transcriptases inverses Monstersript (Epicentre), Omniscript, Sensiscript (Qiagen), les transcriptases inverses ThermoScript et Thermo-X (l'une et l'autre Invitrogen), AccuScript (Stratagene).

11. Procédé selon les revendications 1 à 10 où la réaction comprend en outre une étape de température à une température plus élevée d'environ 65°C à 95°C.

12. Procédé selon les revendications 1 à 11 où l'ADNc généré dans le procédé est ensuite amplifié avec une réaction en chaîne par polymérase et la réaction comprend des amorces aléatoires et/ou des amorces spécifiques et/ou éventuellement une ou plusieurs sondes.

13. Mélange réactionnel comprenant une première enzyme à activité de polyadénylation, une seconde enzyme à activité de transcriptase inverse, des poly-(C)-polynucléotides compétiteurs, éventuellement un tampon, éventuellement au moins un ribonucléotide, éventuellement au moins un désoxyribonucléotide et éventuellement un oligonucléotide d'ancrage, éventuellement des amorces aléatoires et éventuellement une enzyme à activité de synthèse d'ADN.

14. Kit comprenant un mélange réactionnel selon la revendication 13.

15. Utilisation d'un mélange réactionnel comprenant
une première enzyme à activité de polyadénylation, une seconde enzyme à activité de transcriptase inverse, éventuellement un oligonucléotide d'ancrage, éventuellement des poly-(C)-polynucléotides, éventuellement un tampon, éventuellement au moins un ribonucléotide, éventuellement au moins un désoxyribonucléotide et éventuellement des amorces aléatoires, et éventuellement une enzyme à activité de synthèse d'ADN
dans un procédé selon les revendications 1 à 12.
